# EUROPEAN PATENT APPLICATION

(11) **EP 2 428 799 A1**
(43) Date of publication of application: **14.03.2012**
(21) Application number: 11182249.0
(22) Date of filing: 02.11.2007
(51) Int. Cl.: G01N 33/543, G01N 33/58, G01N 33/544

(54) **Magnetic recognition system**

(30) Priority: 02.11.2006 GB 0621894
(62) Divisional of application: 07824427.4
(71) Applicant: ITI Scotland Limited, Glasgow G2 6HQ (GB)
(72) Inventor: Dehal, Prabhjyot, Dundee DD2 1XA (GB); Pritchard, David, Dundee DD2 1XA (GB); Geekie, Claire, Dundee DD2 1XA (GB)
(74) Representative: Hill, Christopher Michael

(57) **Abstract**

Provided is a label for an analyte, which label is attached to a magnetic or magnetisable substance, the label comprising:
(a) a recognition moiety for attaching the label to the analyte; and
(b) a moiety for binding or encapsulating the magnetic or magnetisable substance;

wherein the moiety for binding or encapsulating the magnetic or magnetisable substance comprises a metal-binding protein, polypeptide, or peptide.

SEQUENCE LISTING

<110>  ITI Scotland Limited

<120>  Magnetic Recognition System

<130>  312440EP1/CMH/CJS

<150>  GB0621894.5
<151>  2006-11-02

<160>  7

<170>  PatentIn version 3.1

<210>  1
<211>  590
<212>  PRT
<213>  Artificial Sequence

<220>
<223>  scFv-ferritin fusion protein

<400>  1

<210>  2
<211>  312
<212>  PRT
<213>  Artificial Sequence

<220>
<223>  scFv-MT2 fusion protein

<400>  2

Leu Val Gln Pro Gly Gly Ser Leu Arg Leu Ser Cys Ala Ala Ser Gly
1               5                   10                  15

Phe Thr Phe Ser Ser Phe Ser Met Ser Trp Val Arg Gln Ala Pro Gly
            20                  25                  30

Lys Gly Leu Glu Trp Val Ser Ser Ile Ser Gly Ser Ser Gly Thr Thr
        35                  40                  45

Tyr Tyr Ala Asp Ser Val Lys Gly Arg Phe Thr Ile Ser Arg Asp Asn
    50                  55                  60

Ser Lys Asn Thr Leu Tyr Leu Gln Met Asn Ser Leu Arg Ala Glu Asp
65                  70                  75                  80

Thr Ala Val Tyr Tyr Cys Ala Lys Pro Phe Pro Tyr Phe Asp Tyr Trp
                85                  90                  95

Gly Gln Gly Thr Leu Val Thr Val Ser Ser Gly Asp Gly Ser Ser Gly
            100                 105                 110

Gly Ser Gly Gly Ala Ser Thr Gly Glu Ile Val Leu Thr Gln Ser Pro
        115                 120                 125

Gly Thr Leu Ser Leu Ser Pro Gly Glu Arg Ala Thr Leu Ser Cys Arg
    130                 135                 140

Ala Ser Gln Ser Val Ser Ser Ser Phe Leu Ala Trp Tyr Gln Gln Lys
145                 150                 155                 160

Pro Gly Gln Ala Pro Arg Leu Leu Ile Tyr Tyr Ala Ser Ser Arg Ala
                165                 170                 175

Thr Gly Ile Pro Asp Arg Phe Ser Gly Ser Gly Ser Gly Thr Asp Phe
            180                 185                 190

Thr Leu Thr Ile Ser Arg Leu Glu Pro Glu Asp Phe Ala Val Tyr Tyr
        195                 200                 205

Cys Gln Gln Thr Gly Arg Ile Pro Pro Thr Phe Gly Gln Gly Thr Lys
    210                 215                 220

Val Glu Ile Lys Ser Gly Gly Gly Met Asp Pro Asn Cys Ser Cys Ala

225                 230                 235                 240

Ala Gly Asp Ser Cys Thr Cys Ala Gly Ser Cys Lys Cys Lys Glu Cys
                245                 250                 255

Lys Cys Thr Ser Cys Lys Lys Ser Cys Cys Ser Cys Cys Pro Val Gly
            260                 265                 270

Cys Ala Lys Cys Ala Gln Gly Cys Ile Cys Lys Gly Ala Ser Asp Lys
        275                 280                 285

Cys Ser Cys Cys Ala Pro Gly Ser Ala Gly Gly Ser Gly Gly Asp Ser
    290                 295                 300

Met Ala Glu Val Gln Leu Leu Glu
305                 310

<210>  3
<211>  182
<212>  PRT
<213>  Artificial Sequence

<220>
<223>  Ferritin heavy chain

<400>  3

Met Thr Thr Ala Ser Thr Ser Gln Val Arg Gln Asn Tyr His Gln Asp
1               5                   10                  15

Ser Glu Ala Ala Ile Asn Arg Gln Ile Asn Leu Glu Leu Tyr Ala Ser
            20                  25                  30

Tyr Val Tyr Leu Ser Met Ser Tyr Tyr Phe Asp Arg Asp Asp Val Ala
        35                  40                  45

Leu Lys Asn Phe Ala Lys Tyr Phe Leu His Gln Ser His Glu Glu Arg
    50                  55                  60

Glu His Ala Lys Leu Met Lys Leu Gln Asn Gln Arg Gly Gly Arg Ile
65                  70                  75                  80

Phe Leu Gln Asp Ile Lys Lys Pro Asp Cys Asp Asp Trp Glu Ser Gly
                85                  90                  95

Leu Asn Ala Met Glu Cys Ala Leu His Leu Glu Lys Asn Val Asn Gln
            100                 105                 110

Ser Leu Leu Glu Leu His Lys Leu Ala Thr Asp Lys Asn Asp Pro His
        115                 120                 125

Leu Cys Asp Phe Ile Glu Thr His Tyr Leu Asn Glu Gln Val Lys Ala
    130                 135                 140

Ile Lys Glu Leu Gly Asp His Val Thr Asn Leu Arg Lys Met Gly Ala
145                 150                 155                 160

Pro Glu Ser Gly Leu Ala Glu Tyr Leu Phe Asp Lys His Thr Leu Gly
                165                 170                 175

Asp Ser Asp Asn Glu Ser
            180

<210>  4
<211>  175
<212>  PRT
<213>  Artificial Sequence

<220>
<223>  Ferritin light chain

<400>  4

<210>  5
<211>  588
<212>  PRT
<213>  Artificial Sequence

<220>
<223>  Ferritin fusion protein

<400>  5

Leu Val Gln Pro Gly Gly Ser Leu Arg Leu Ser Cys Ala Ala Ser Gly
1               5                   10                  15

Phe Thr Phe Ser Ser Phe Ser Met Ser Trp Val Arg Gln Ala Pro Gly
            20                  25                  30

Lys Gly Leu Glu Trp Val Ser Ser Ile Ser Gly Ser Ser Gly Thr Thr
        35                  40                  45

Tyr Tyr Ala Asp Ser Val Lys Gly Arg Phe Thr Ser Arg Asp Asn Ser
    50                  55                  60

Lys Asn Thr Leu Tyr Leu Gln Met Asn Ser Leu Arg Ala Glu Asp Thr
65                  70                  75                  80

Ala Val Tyr Tyr Cys Ala Lys Pro Phe Pro Tyr Phe Asp Tyr Trp Gly
                85                  90                  95

Gln Gly Thr Leu Val Thr Val Ser Ser Gly Asp Gly Ser Ser Gly Gly
            100                 105                 110

Ser Gly Gly Ala Ser Thr Gly Glu Ile Val Leu Thr Gln Ser Pro Gly
        115                 120                 125

Thr Leu Ser Leu Ser Pro Gly Glu Arg Ala Thr Leu Ser Cys Arg Ala
    130                 135                 140

Ser Gln Ser Val Ser Ser Ser Phe Leu Ala Trp Tyr Gln Gln Lys Pro
145                 150                 155                 160

Gly Gln Ala Pro Arg Leu Leu Ile Tyr Tyr Ala Ser Ser Arg Ala Thr
                165                 170                 175

Gly Ile Pro Asp Arg Phe Ser Gly Ser Gly Ser Gly Thr Asp Phe Thr
            180                 185                 190

Leu Thr Ile Ser Arg Leu Glu Pro Glu Asp Phe Ala Val Tyr Tyr Cys
        195                 200                 205

Gln Gln Thr Gly Arg Ile Pro Pro Thr Phe Gly Gln Gly Thr Lys Val
    210                 215                 220

Glu Ile Lys Ser Gly Gly Gly Met Thr Thr Ala Ser Thr Ser Gln Val
225                 230                 235                 240

Arg Gln Asn Tyr His Gln Asp Ser Glu Ala Ala Ile Asn Arg Gln Ile
                245                 250                 255

Asn Leu Glu Leu Tyr Ala Ser Tyr Val Tyr Leu Ser Met Ser Tyr Tyr
            260                 265                 270

Phe Asp Arg Asp Asp Val Ala Leu Lys Asn Phe Ala Lys Tyr Phe Leu
        275                 280                 285

His Gln Ser His Glu Glu Arg Glu His Ala Lys Leu Met Lys Leu Gln
    290                 295                 300

Asn Gln Arg Gly Gly Arg Ile Phe Leu Gln Asp Ile Lys Lys Pro Asp
305                 310                 315                 320

Cys Asp Asp Trp Glu Ser Gly Leu Asn Ala Met Glu Cys Ala Leu His
                325                 330                 335

Leu Glu Lys Asn Val Asn Gln Ser Leu Leu Glu Leu His Lys Leu Ala
            340                 345                 350

Thr Asp Lys Asn Asp Pro His Leu Cys Asp Phe Ile Glu Thr His Tyr
        355                 360                 365

Leu Asn Glu Gln Val Lys Ala Ile Lys Glu Leu Gly Asp His Val Thr
    370                 375                 380

Asn Leu Arg Lys Met Gly Ala Pro Glu Ser Gly Leu Ala Glu Tyr Leu
385                 390                 395                 400

Phe Asp Lys His Thr Leu Gly Asp Ser Asp Asn Glu Ser Met Ser Ser
                405                 410                 415

Gln Ile Arg Gln Asn Tyr Ser Thr Asp Val Glu Ala Ala Val Asn Ser
            420                 425                 430

Leu Val Asn Leu Tyr Leu Gln Ala Ser Tyr Thr Tyr Leu Ser Leu Gly
        435                 440                 445

Phe Tyr Phe Asp Arg Asp Asp Val Ala Leu Glu Gly Val Ser His Phe
    450                 455                 460

Phe Arg Glu Leu Ala Glu Glu Lys Arg Glu Gly Tyr Glu Arg Leu Leu
465                 470                 475                 480

Lys Met Gln Asn Gln Arg Gly Gly Arg Ala Leu Phe Gln Asp Ile Lys
                485                 490                 495

Lys Pro Ala Glu Asp Glu Trp Gly Lys Thr Pro Asp Ala Met Lys Ala
            500                 505                 510

Ala Met Ala Leu Glu Lys Lys Leu Asn Gln Ala Leu Leu Asp Leu His
        515                 520                 525

Ala Leu Gly Ser Ala Arg Thr Asp Pro His Leu Cys Asp Phe Leu Glu
    530                 535                 540

Thr His Phe Leu Asp Glu Glu Val Lys Leu Ile Lys Lys Met Gly Asp
545                 550                 555                 560

His Leu Thr Asn Leu His Arg Leu Gly Gly Pro Glu Ala Gly Leu Gly
                565                 570                 575

Glu Tyr Leu Phe Glu Arg Leu Thr Leu Lys His Asp
            580                 585

<210>  6
<211>  80
<212>  PRT
<213>  Artificial Sequence

<220>
<223>  MT2

<400>  6

Met Asp Pro Asn Cys Ser Cys Ala Ala Gly Asp Ser Cys Thr Cys Ala
1               5                   10                  15

Gly Ser Cys Lys Cys Lys Glu Cys Lys Cys Thr Ser Cys Lys Lys Ser
            20                  25                  30

Cys Cys Ser Cys Cys Pro Val Gly Cys Ala Lys Cys Ala Gln Gly Cys
        35                  40                  45

Ile Cys Lys Gly Ala Ser Asp Lys Cys Ser Cys Cys Ala Pro Gly Ser
    50                  55                  60

Ala Gly Gly Ser Gly Gly Asp Ser Met Ala Glu Val Gln Leu Leu Glu
65                  70                  75                  80

<210>  7
<211>  312
<212>  PRT
<213>  Artificial Sequence

<220>
<223>  MT2 fusion protein

<400>  7

Leu Val Gln Pro Gly Gly Ser Leu Arg Leu Ser Cys Ala Ala Ser Gly
1               5                   10                  15

Phe Thr Phe Ser Ser Phe Ser Met Ser Trp Val Arg Gln Ala Pro Gly
            20                  25                  30

Lys Gly Leu Glu Trp Val Ser Ser Ile Ser Gly Ser Ser Gly Thr Thr
        35                  40                  45

Tyr Tyr Ala Asp Ser Val Lys Gly Arg Phe Thr Ile Ser Arg Asp Asn
    50                  55                  60

Ser Lys Asn Thr Leu Tyr Leu Gln Met Asn Ser Leu Arg Ala Glu Asp
65                  70                  75                  80

Thr Ala Val Tyr Tyr Cys Ala Lys Pro Phe Pro Tyr Phe Asp Tyr Trp
                85                  90                  95

Gly Gln Gly Thr Leu Val Thr Val Ser Ser Gly Asp Gly Ser Ser Gly
            100                 105                 110

Gly Ser Gly Gly Ala Ser Thr Gly Glu Ile Val Leu Thr Gln Ser Pro
        115                 120                 125

Gly Thr Leu Ser Leu Ser Pro Gly Glu Arg Ala Thr Leu Ser Cys Arg
    130                 135                 140

Ala Ser Gln Ser Val Ser Ser Ser Phe Leu Ala Trp Tyr Gln Gln Lys
145                 150                 155                 160

Pro Gly Gln Ala Pro Arg Leu Leu Ile Tyr Tyr Ala Ser Ser Arg Ala
                165                 170                 175

Thr Gly Ile Pro Asp Arg Phe Ser Gly Ser Gly Ser Gly Thr Asp Phe
            180                 185                 190

Thr Leu Thr Ile Ser Arg Leu Glu Pro Glu Asp Phe Ala Val Tyr Tyr
        195                 200                 205

Cys Gln Gln Thr Gly Arg Ile Pro Pro Thr Phe Gly Gln Gly Thr Lys
    210                 215                 220

Val Glu Ile Lys Ser Gly Gly Gly Met Asp Pro Asn Cys Ser Cys Ala
225                 230                 235                 240

Ala Gly Asp Ser Cys Thr Cys Ala Gly Ser Cys Lys Cys Lys Glu Cys
                245                 250                 255

Lys Cys Thr Ser Cys Lys Lys Ser Cys Cys Ser Cys Cys Pro Val Gly
            260                 265                 270

Cys Ala Lys Cys Ala Gln Gly Cys Ile Cys Lys Gly Ala Ser Asp Lys
        275                 280                 285

Cys Ser Cys Cys Ala Pro Gly Ser Ala Gly Gly Ser Gly Gly Asp Ser
    290                 295                 300

Met Ala Glu Val Gln Leu Leu Glu
305                 310

## Description

The present invention concerns magnetic recognition labels, capable of attaching small quantities of a magnetic (or magnetisable) substance to an analyte via a recognition agent for the analyte. The labels have significant advantages in that they are capable of attaching a very small volume of the magnetic substance to the analyte, so that the analyte can be influenced by magnetic fields, even in a confined space, such as in a microfluidic system. The presence of the magnetic substance allows more sophisticated spatial manipulation of the analyte, which is particularly beneficial in a microfluidic system. The invention also concerns products, methods and uses relating to the labels.

It is well known that magnetic beads may be employed to control molecules that are involved in assay methods (see, for example, US2006/084089). Typically, such beads are attached to a molecule (such as an antibody) that can recognise and bind the analyte. The magnetic properties of the beads are employed to control or spatially manipulate the analyte, e.g. to separate the analyte from other molecules in a sample.

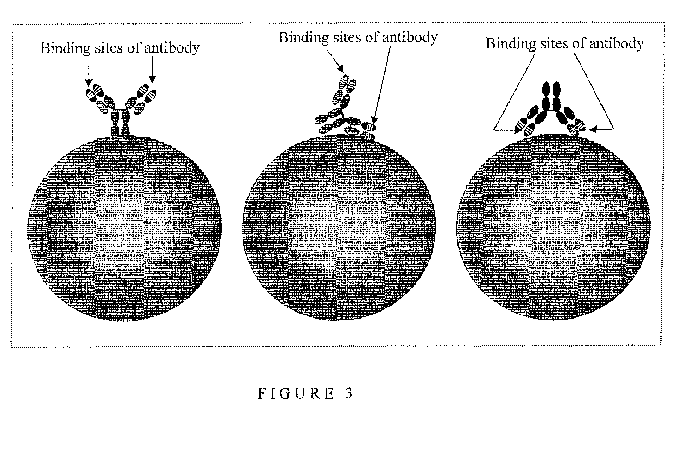

However, magnetic beads are not suitable for all systems. More recently it has become possible to work with ever-smaller quantities of sample using microfluidic or nanofluidic devices. Such devices are capable of assaying for particular substances in a very small sample, such as a drop of blood from a pin-prick. The dimensions of the channels in such devices may often be too small to accommodate magnetic beads satisfactorily, even though such beads can be made on the micrometer scale, either because they are larger than the channels, or because they give rise to clogging, or blockages in the channels. This is described further at www.deas.harvard.edu/projects/weitzlab/wyss.preprint.2006.pdf. Although small beads have a large surface area to volume ratio (Table 1), particularly small beads or particles can suffer from steric hindrance where an attached protein blocks the attachment of another protein. This is made particularly problematic by the random spatial organisation of antibodies, or other recognition entities, exhibited on attaching them to a particle. This is further exacerbated, because when coupling a protein to the surface of a magnetic bead or particle, the required orientation of the protein may not be optimal (see Figure 3).

**Table 1. A comparison of the surface area to volume ratio of 3µm and 50nm beads.**

| | Surface Area (SA) (4πr²) | Vol ([4/3] πr³) | Ratio SA:Vol |
|---|---|---|---|
| 3 µm bead | 4 π (1.5)² | (4/3) x π x (1.5)³ | 2:1 |
| | = 28 µm² | = 14 µm³ | |
| 50nm bead | 4 π(0.025)² | (4/3)π(0.025)³ | 120:1 |
| (or 0.05µm) | = 7.8 x 10⁻³ µm² | =6.5 x 10⁻⁵ µm³ | |

Attempts have been made to bind smaller magnetic particles to proteins, although this has not yet received much attention for microfluidic and nanofluidic purposes. For example, published PCT application WO 2006/104700 describes magnetic protein nanosensors which may be used in arrays for detecting analytes in a liquid sample. In this system, a fusion protein is employed, typically comprising a T4 tail-fibre gene modified to contain additional functional groups (for example peptide display ligands) which will bind paramagnetic nanoparticles.

Similarly, WO 2004/083902 discloses magnetic nanoparticles probes for intracellular magnetic imaging. The probes are typically formed from self-assembled coating materials surrounding the magnetic material, such as micelles, liposomes or dendrimers. The surface of the encapsulated magnetic particles may be attached to a delivery ligand, such as a peptide. An analogous system is disclosed in US 5,958,706, which concerns magnetic particles encapsulated within an organic membrane (such as a phospholipid membrane) and attached to a membrane protein. In Tomoko Yoshino et al. "Efficient and stable display of functional proteins on bacterial magnetic particles using mms13 as a novel anchor molecule", Applied and Environmental Microbiology Jan. 2006, p. 465-471, a method of protein display on bacterial magnetic particles is disclosed. The magnetic particles are also covered with a lipid bilayer membrane and novel mms13 protein binds to the particles.

Some work has also been carried out on viral encapsulation of magnetic nanoparticles. US 2006/0240456 discloses encapsulation of magnetic cobalt within the viral capsid protein shell of a T7 bacteriophage.

In a separate development, it had been discovered that some proteins have the ability to bind directly to metal ions. Meldrum F.C. et al. have reported such proteins in Science, 257(5069) 522-3, 1992 "Magnetoferritin: *in vitro* synthesis of a novel magnetic protein". Other work in this field includes Martinez, J. S., et al. 2000. "Self-Assembling Amphiphilic Siderophores from Marine Bacteria.", Science 287 1245-47. More remote work includes the following:
Zborowski et al. 1996. "Immunomagnetic isolation of magnetoferritin-labelled cells in a modified ferrograph." Cytometry 24:251-259 disclose that biotinylated antibodies against various cellular targets and biotinylated magnetoferritin were coupled using an avidin bridge.

Further disclosures in this field include: Inglis, et al. 2004. "Continuous microfluidic immunomagnetic cell separation." Applied physics letters. 85 (21) 5093-5; Inglis et al. 2006. "Microfluidic high gradient magnetic cell separation." Journal of Applied Physics 99; Lambert et al. 2005. "Evolution of the transferrin family: Conservation of residues associated with iron and anion binding." Comparative Biochem and Physiol, (B) 142 129 - 141; Gider et al. 1995. "Classical and quantum magnetic phenomena in natural and artificial ferritin proteins." Science. 268 77-80; Haukanes, B.I. and Kvam, C. 1993. "Application of magnetic beads in bioassays." Biotechnology (N Y). 11 (1) 60-3; Olsvik, O. et al. 1994. "Magnetic separation techniques in diagnostic microbiology." Clin Microbiol Rev. 7 (1) 43-54; Archer, M.J. et al. 2006, "Magnetic bead-based solid phase for selective extraction of genomic DNA." Anal Biochem. doi:10.1016/j.ab.2006.05.005; Schneider, T. et al. 2006. "Continuous flow magnetic cell fractionation based on antigen expression level." J Biochem Biophys Methods. 68 (1) 1-21; Ramadan, Q. et al. 2006, "An integrated microfluidic platform for magnetic microbeads separation and confinement." Biosens Bioelectron. 21 (9) 1693-702; Cotter, M.J., et al. 2001. « A novel method for isolation of neutrophils from murine blood using negative immunomagnetic separation." Am J Pathol. 159 (2) 473-81; http://www.newscientist.com/article.ns?id=dn3664; and Chang, C.C., et al. 2006, "Mn,Cd-metallothionein-2: a room temperature magnetic protein." Biochem Biophys Res Commun. 340 (4) 1134-8. In this latter article, the small ion binding protein metallothionein-2 (MT) was manipulated to bind cadmium and manganese rather than zinc, thereby rendering the protein magnetic.

Further background disclosure includes: Odette et al. 1984, "Ferritin conjugates as specific magnetic labels." Biophys. J. 45 1219-22; Yamamoto et al. 2002, "An iron-binding protein, Dpr, from Streptococcus mutans prevents iron-dependent hydroxyl radical formation in vitro." J Bacteriol. 184 (11) 2931-9; and Ishikawa et al. 2003, "The iron-binding protein Dps confers hydrogen peroxide stress resistance to Campylobacter jejuni. " J Bacteriol. 185 (3) 1010-17.

CA 2,521,639 discloses the use of ferritin to remove contaminant ions from solution. The ferritin forms part of a larger structure which also contains other ion-exchange species (e.g. porphyrins or crown-ether). The other ion-exchange species are designed to remove the contaminant, whilst the magnetic properties of the ferritin are employed to remove the species from the solution.

United States Patent 7,097,841 discloses ferritin fusion proteins for use in vaccines amongst other applications.

Despite the extensive disclosure on magnetic particles and nanoparticles set out above, there is an ongoing requirement for more simple and effective magnetic particle labels for use in microfluidic and nanofluidic systems.

It is an aim of the present invention to solve the above problems and improve on known products and methods, such as those outlined above. It is a further aim of the present invention to provide an improved label for an analyte that could be advantageously employed, for example, in a microfluidic or nanofluidic device. It is a further aim of the present invention to provide methods, kits and uses employing such labels.

Accordingly, the present invention provides a label for an analyte, which label is attached to a magnetic or magnetisable substance, the label comprising:
(a) a recognition moiety for attaching the label to the analyte; and
(b) a moiety for binding or encapsulating the magnetic or magnetisable substance;
wherein the moiety for binding or encapsulating the magnetic or magnetisable substance comprises a metal-binding protein, polypeptide, or peptide.

The inventors have surprisingly determined that quantities of a magnetic or magnetisable substance small enough to be useful in microfluidic and/or nanofluidic devices, can be attached to an analyte of choice by incorporating metal atoms or ions (or compounds containing them) in a metal-binding protein, polypeptide, or peptide that is attached to a recognition agent that can in turn attach to the analyte. Thus, the labels of the present invention comprise at least two moieties: a recognition moiety for attaching the label to the desired analyte, and a moiety for binding the magnetic or magnetisable substance. The labels are simple to purify using established techniques, such as affinity purification, or magnetic field purification.

When the recognition moieties are of single valency, they avoid problems arising from cross-linking of receptors on cell surfaces (unlike antibodies). The inventors have also overcome 'clogging' problems encountered in known methods by coupling targeting proteins directly (or indirectly) to magnetisable proteins using established molecular biology strategies.

The labels of the present invention have the further advantage that they may be magnetised or de-magnetised using simple chemical procedures.

It is particularly preferred that the labels are fusion proteins. In the context of the present invention, a fusion protein is a protein that has been expressed as a single entity recombinant protein. Fusion proteins have a number of further advantages. The orientation of the recognition arm of the fusion protein (e.g. the scFv) within the invention will be controlled and therefore more likely to bind its target. Fusion proteins also facilitate the possibility of incorporating a plurality of recognition moieties in a single fusion protein. These recognition sites may be directed against the same target or to different targets. Where two or more recognition moieties are present, the spatial organisation of the recognition moieties on the magnetic substance can be defined and controlled, decreasing problems caused by steric hindrance and random binding to conventional beads. With careful spacing of each recognition moiety within the fusion protein (e.g. by incorporating nucleic acid spacers in the expression system) the tertiary structure of the final protein can be controlled to deploy recognition moieties at spatially selected zones across the protein surface. A further advantage of using fusion proteins is that the number of recognition moieties within each label can be specified and will be identical for every molecule of the label. This contrasts with conventional means of attaching recognition moieties to magnetic beads, where due to the random nature of attachment it is much more difficult to specify the number of recognition moieties and there will be considerable variation in the number that are attached to each magnetic bead.

By 'attached to' in the present context, it is meant that the attachment is of any type, including specific and non-specific binding and also encapsulation. Thus, the moiety for binding the magnetic or magnetisable substance should be capable of binding or encapsulating (or otherwise attaching in a specific or non-specific manner) the substance in the form of particles or aggregates or the like. These particles or aggregates are much smaller than conventional magnetic beads, typically having less than 100,000 atoms, ions or molecules, more preferably less than 10,000 atoms, ions or molecules, and most preferably less than 5,000 atoms ions or molecules bound or encapsulated to the (or each) moiety in total. The most preferred substances are capable of binding up to 3,000 atoms ions or molecules, and in particular approximately 2,000 or less, or 500 or less such species.

In one specific example employed in the invention, the metallic component of ferritin (a 24 subunit protein shell) consists of an 8 nm (8x10⁻⁹ m) inorganic core. Each core contains approximately 2,000 Fe atoms. In another example, Dpr, from *Streptococcus mutans* (a 12 subunit shell), consists of a 9 nm shell containing 480 Fe atoms. In a further example, lactoferrin binds 2 Fe atoms and contains iron bound to haem (as opposed to ferritin which binds iron molecules within its core). Metallothionein-2 (MT) binds 7 divalent transition metals. The zinc ions within MT are replaced with Mn²⁺ and Cd²⁺ to create a room temperature magnetic protein. MT may be modified to further incorporate one or more additional metal binding sites, which increases the magnetism of the Mn, Cd MT protein.

In accordance with these binding environments, the total volume of the substance bound or encapsulated in a single moiety typically does not exceed 1x10⁵ nm³ (representing a particle or aggregate of the substance having an average of about 58 nm or less). More preferably the substance may have a total volume of not more than 1x10⁴ nm³ (representing a particle or aggregate of the substance having an average diameter of about 27 nm or less). More preferably still the substance may have a total volume of not more than 1x10³ nm³ (representing a particle or aggregate of the substance having an average diameter of about 13 nm or less). Most preferably the substance may have a total volume of not more than 100 nm³ (representing a particle or aggregate of the substance having an average diameter of 6 nm or less). However, the size of the particles may be determined by average diameter as an alternative to volume. It is thus also preferred in the present invention that the average diameter of the bound particles is 50 nm or less, 40 nm or less, 30 nm or less, 20 nm or less or most preferably 10 nm or less. In this context, average means the sum of the diameters of the number of particles, divided by the number of particles.

The present invention will now be described in more detail by way of example only with reference to the following Figures:

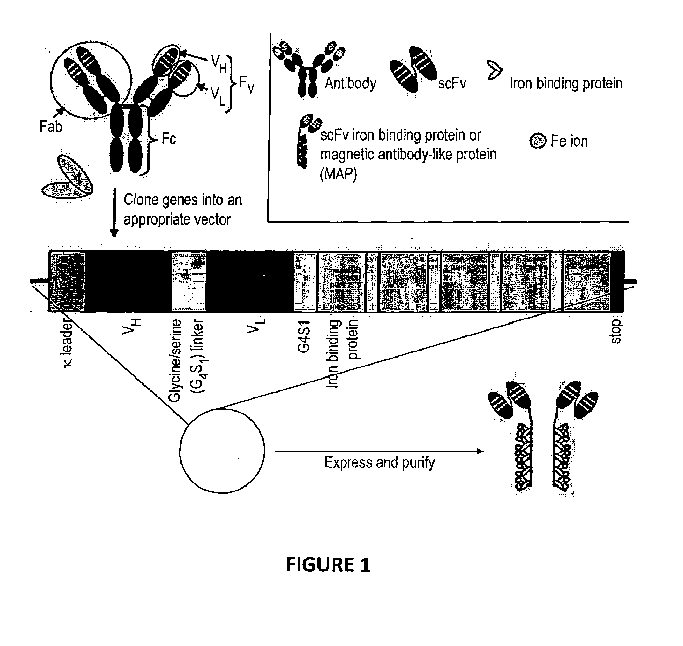

Figure 1: this Figure shows how the appropriate genes are cloned into a vector in order to produce the labels of the present invention. The number of magnetisable protein units in the final label may be controlled by including as many copies of the appropriate gene as necessary. Only genes for the V_{H} and V_{L} regions of the antibody are included in this example, so that the scFv portion of the antibody is included in the final preferred chimaeric protein, rather than the full antibody.
   The V_{H} and V_{L} regions can be cloned by amplification of the appropriate genes (messenger RNA) using reverse transcription followed by the polymerase chain reaction (PCR) from monoclonal hybridoma clones, or (phage display) gene libraries into an appropriate expression vector. The genes are linked by a series of small amino acids (for example, four glycine and one serine residues) to allow for the correct alignment of the polypeptides relative to each other and the formation of the binding site without interference from the linker region. The gene(s) for the magnetisable protein are then cloned either directly after the scFv or separated by an amino acid linker as above. If necessary, a purification tag (such as hexahistidine, glutathione-s-transferase, b-galactosidase, haemaglutinin, green fluorescent protein, etc.) can be incorporated to aid in the isolation of the fusion protein. A stop codon is incorporated into the end of the fusion protein's gene, followed by a polyadenylation site. If the magnetisable protein chosen is composed of multiple subunits (such as ferritin or Dpr), it is envisaged that the genes encoding these subunits would follow or precede the scFv. It may also be desirable to incorporate the scFv genes within the genes of the magnetisable protein, to locate the scFv amino acid sequence on a conveniently located portion of the magnetisable protein that is not at the amino or carboxyl terminus. If a monomeric protein is chosen (such as MT), multiple copies of the scFv and or metal binding moiety genes can be cloned in tandem into the expression vector (as in Figure 1). The position of the scFv and metal binding moieties within the expressed fusion protein can be defined and controlled by the genetic sequence. As for multimeric proteins, it may be desirable to incorporate the scFv genes within the genes of the magnetisable protein, to locate the scFv amino acid sequence on a conveniently located portion of the magnetisable protein that is not at the amino or carboxyl terminus. The vector is then introduced into an expression system such as a mammalian or insect cell line, or a yeast or bacterial host for expression. The fusion protein is harvested by appropriate methods (sedimentation, immunoprecipitation, affinity purification, high performance or fast protein liquid chromatography, etc.). The purified fusion protein is then modified using established methods to magnetise the protein (Chang *et al.,* Meldrum *et al*.).

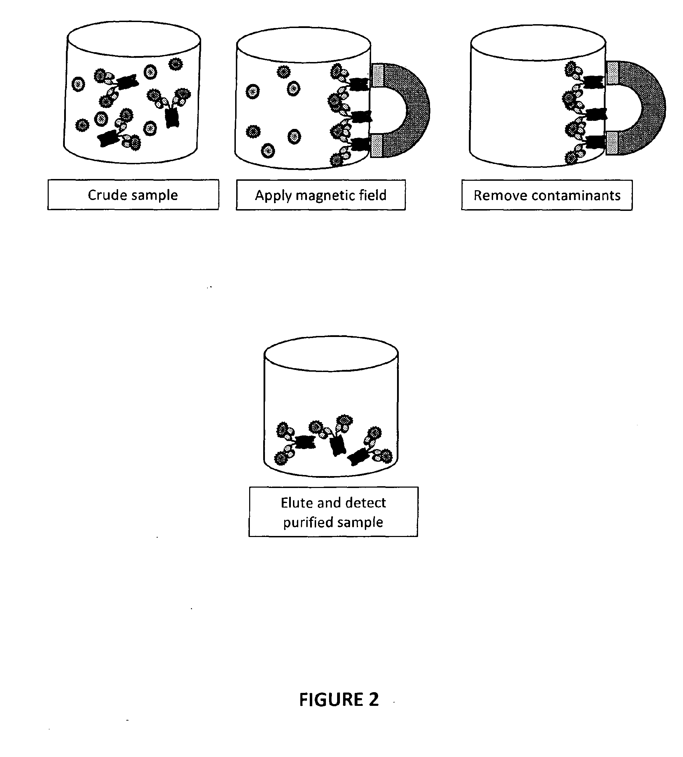

Figure 2: this Figure shows a schematic purification method using the labels of the present invention. The analyte of interest is labelled using the labels of the present invention, which are bound to ions. A magnetic field is applied to prevent the bound analyte from being washed away, whilst all contaminants are removed. The purified sample is left, which may be analysed (e.g. detected) if desired.
Figure 3: This Figure shows that, since currently available commercial antibody-coated beads are manufactured by covalently conjugating antibodies to beads, there is a possibility of incorrectly orientating the antibodies, thereby reducing the efficiency of binding.

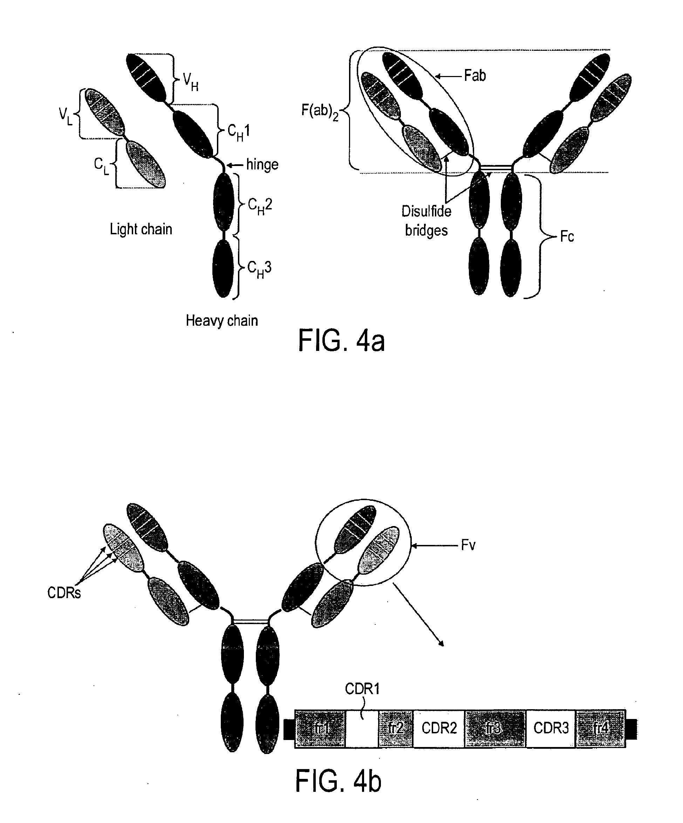

Figure 4a and 4b: these Figures schematically depict a simplification of the structure of antibodies such as IgG. After protease treatment using enzymes such as papain, antibodies are split into 3 parts close to the hinge region. As the effector function part of antibodies (the hinge, C_{H}2 and C_{H}3) are relatively easy to crystallise for X-ray diffraction analysis, this part has become known as the crystallisable fragment (Fc) region. The antigen binding portions of antibodies are known as the antibody fragment (Fab). After enzymic digestion, the Fab fragments can be linked at the hinge region thereby forming a F(ab)₂ fragment. Other antibodies may have differences in the number of domains in the Fc region and variations in the hinge region.
Figures 5a and 5b: these Figures show the construction of a scFv-ferritin fusion protein.
Figures 6a and 6b: these Figures show the construction of a scFv-MT2 fusion protein.
Figure 7: this Figure shows the construction of a scFv fragment.
Figure 8: this Figure shows construction of a cDNA library. In order to construct a cDNA library from a tissue sample, mRNA is extracted, reverse transcribed into cDNA and ligated into plasmid vectors. These vectors are then used to transform bacteria cells. The transformed cells are stored frozen until required. The frozen cells can be expanded by growing in appropriate media and the plasmids purified. Genes of interest can then be PCR amplified for further analysis using specific primer pairs.
Figures 9a and 9b: these Figures show PCR amplicons of the ferritin heavy (H) and light (L) chain genes, and the overlapped PCR product of ferritin heavy and light chain genes, respectively. Figure 9c shows colony PCR results, clones 1, 3 and 4 were selected for sequencing.
Figures 10a and 10b: these Figures show a gel showing the products of a PCR amplification of the anti-fibronectin scFv and ferritin heavy and light polygene (arrowed), and a gel showing the overlap PCR products, respectively.
Figure 11: this Figure shows a gel showing the results of a PCR screen of a number of clones transformed using plasmids that had been ligated with the scFv:ferritin fusion constructs.
Figure 12: this Figure shows Coomassie blue stained gel and Western blot of cell lysates respectively. Key: 1. Ferritin 2 hour induction; 2. Ferritin 3 hour induction; 3. Ferritin 4 hour induction; 4. Benchmark (Invitrogen) Protein Ladder.
Figure 13: this Figure shows a gel showing the PCR amplification product of MT2 from a human liver library.
Figure 14: this Figure shows colony analysis of clones transformed with plasmid containing the scFv:MT2 construct.
Figure 15: this Figure shows (respectively) Coomassie gel and western blot of scFv:MT2 (arrowed).
Figure 16: this Figure shows photographs of a Coomassie blue stained gel and western blot (respectively) of the re-solubilised scFv:ferritin and scFv:MT2 fusion proteins. The fusion proteins are circled - ferritin is in lane 2 on both gels and MT2 is in lane 3 of both gels. A protein molecular weight ladder is in lane 1.
Figure 17a and 17b: these Figures show overlaid Sensograms from the SPR analysis of the binding of MT2 and ferritin fusion proteins respectively.
Figure 18: this Figure demonstrates the magnetic nature of the magnetoferritin produced for use in the present invention.

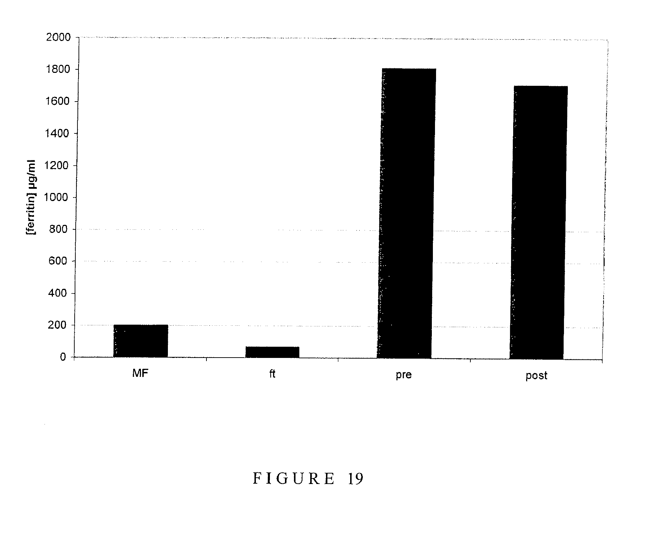

Figure 19: this Figure shows the concentration of ferritin during the production and concentration of magnetoferritin. Key: MF; Magnetoferritin: ft; Flow-through: Pre; pre-dialysis Macs® column concentrated magnetoferritin: post; post-dialysis Macs® column concentrated magnetoferritin.
Figure 20: this Figure shows binding of scFv:ferritin and heat treated scFv:ferritin to fibronectin.
Figures 21a and 21b: these Figures show absorbance measurements, recorded using a Varioskan Flash instrument, on magnetised fusion protein. After concentration the protein is still recognised by the monoclonal anti-ferritin antibody (21a) and the magnetised anti-fibronectin ferritin fusion protein retains binding ability to its target antigen (21b).

The moiety for binding the magnetic or magnetisable substance is not especially limited, provided that it is capable of binding the substance and does not interfere with the binding to the analyte. The moiety for binding the magnetic or magnetisable substance comprises a metal-binding protein, polypeptide or peptide (or the metal-binding domain of such a protein polypeptide or peptide). Typically this moiety is capable of binding to, or is bound to, one or more transition and/or lanthanide metal atoms and/or ions, or any compound comprising such ions. Such ions include, but are not limited to, any one or more ions of Fe, Co, Ni, Mn, Cr, Cu, Zn, Cd, Y, Gd, Dy, or Eu.

In the more preferred embodiments of the invention, the one or more metal ions comprise any one or more of Fe2⁺, Fe³⁺, Co²⁺, Co³⁺, Mn²⁺, Mn³⁺, Mn⁴⁺, Cd²⁺ and Ni²⁺. The most preferred ions for use in the present invention are Fe²⁺ and Fe³⁺ and Cd²⁺ and Mn²⁺ions. Typically these ions are bound by lactoferrin, transferrin and ferritin in the case of iron, and metallothionein-2 in the case of cadmium and manganese. The binding of Fe²⁺ is preferably promoted by employing acidic conditions, whilst the binding of Fe³⁺ is preferably promoted by employing neutral or alkaline conditions.

In preferred embodiments of the invention, the metal-binding moiety comprises a protein, or a metal-binding domain of a protein, selected from lactoferrin, transferrin, ferritin (apoferritin), a metallothionein (MT1 or MT2), a ferric ion binding protein (FBP e.g. from Haemophilus influenzae), frataxin and siderophores (very small peptides which function to transport iron across bacterial membranes).

In some embodiments, the labels of the invention may comprise a plurality of moieties for binding the magnetic or magnetisable substance. The number of such moieties may be controlled so as to control the magnetic properties of the label. Typically in such embodiments, the labels may comprise from 2-100 such moieties, preferably from 2-50 such moieties and most preferably from 2-20 such moieties for binding the magnetic or magnetisable substance. In the final chimaeric protein, each copy of the metal-binding protein may be attached to the next by non-charged amino acid linker sequences for flexibility.

Sets of labels, each label in the set having a different (unique) number of metal-binding moieties are also included in the invention. These sets of labels are advantageous because they may allow processing of multiple analytes from one or more samples simultaneously.

The recognition moiety is not especially limited, provided that it is capable of binding to the analyte of interest. Typically, the analytes to which the moiety should bind are selected from a biological molecule (natural or synthetic), an infectious agent or component of an infectious agent (such as a virus or virus particle or virus component), a cell or cellular component, and a small molecule such as an endogenous or exogenous small molecule (e.g. a metabolite, or a pharmaceutical or drug). In the current context a small molecule means a molecular chemical such as a biologically active molecule that is not a polymer or an oligomer (unlike a protein nucleic acid, polypeptide, or other biological oligomers and polymers), such as a metabolite, a pharmaceutical, a drug, a carbohydrate, a lipid, a fat or the like. Typically a small molecule has a mass of 2,000 Daltons or less. More specifically, it is preferred that the analytes to which the moiety should bind comprise a virus or virus particle or virus component, a protein, a polypeptide, a glycoprotein, a nucleic acid, such as DNA or RNA, an oligonucleotide, a metabolite, a carbohydrate such as a complex carbohydrate, a lipid, a fat, or a pharmaceutical or drug. These analytes include sugar residues produced by bacteria (e.g. sialic acid) and sugar coats on many bacteria/viruses, as well as altered sugars present in some tumours on their glycoproteins. Any one or more of these analytes are preferred for use with the methods of the present invention.

The recognition moiety that is capable of binding to the above analytes may itself be any type of substance or molecule, provided that it is suitable for binding to an analyte of interest. Generally, the recognition moiety is selected from an antibody or a fragment of an antibody, a receptor or a fragment of a receptor, a protein, a polypeptide, a peptidomimetic, a nucleic acid, an oligonucleotide and an aptamer. In more preferred embodiments of the invention, the recognition moiety is selected from a variable polypeptide chain of an antibody (Fv), a T-cell receptor or a fragment of a T-cell receptor, avidin, and streptavidin. Most preferably, the recognition moiety is selected from a single chain of a variable portion of an antibody (sc-Fv).

Antibodies are immunoglobulin molecules involved in the recognition of foreign antigens and expressed by vertebrates. Antibodies are produced by a specialised cell type known as a B-lymphocyte or a B-cell. An individual B-cell produces only one kind of antibody, which targets a single epitope. When a B-cell encounters an antigen it recognises, it divides and differentiates into an antibody producing cell (or plasma cell).

The basic structure of most antibodies is composed of four polypeptide chains of two distinct types (Figure 4). The smaller (light) chain being of molecular mass 25 kilo-Daltons (kDa) and a larger (heavy) chain of molecular mass 50-70 kDa. The light chains have one variable (V_{L}) and one constant (C_{L}) region. The heavy chains have one variable (V_{H}) and between 3-4 constant (C_{H}) regions depending on the class of antibody. The first and second constant regions on the heavy chain are separated by a hinge region of variable length. Two heavy chains are linked together at the hinge region via disulfide bridges. The heavy chain regions after the hinge are also known as the Fc region (crystallisable fragment). The light chain and heavy chain complex before the hinge is known as the Fab (antibody fragment) region, with the two antibody binding sites together known as the F(ab)₂ region. The constant regions of the heavy chain are able to bind other components of the immune system including molecules of the complement cascade and antibody receptors on cell surfaces. The heavy and light chains of antibodies form a complex often linked by a disulfide bridge, which at the variable end is able to bind a given epitope (Figure 4).

The variable genes of antibodies are formed by mutation, somatic recombination (also known as gene shuffling), gene conversion and nucleotide addition events.

ScFv antibodies may be generated against a vast number of targets including:
1. Viruses: Torrance et al. 2006. Oriented immobilisation of engineered single-chain antibodies to develop biosensors for virus detection. J Virol Methods. 134 (1-2) 164-70.
2. Hepatitis C virus: Gal-Tanamy et al. 2005. HCV NS3 serine protease-neutralizing single-chain antibodies isolated by a novel genetic screen. J Mol Biol. 347 (5):991-1003), and Li and Allain. 2005. Chimeric monoclonal antibodies to hypervariable region 1 of hepatitis C virus. J Gen Virol. 86 (6) 1709-16.
3. Cancers: Holliger and Hudson. Engineered antibody fragments and the rise of single domains. Nat Biotechnol. 23 (9) 1126-36.
and may be used in various applications including proteomics (Visintin et al. 2004. Intracellular antibodies for proteomics. J lmmunol Methods. 290 (1-2):135-53).

Thus, in its most preferred embodiments, the present invention makes use of a multi-moiety label, typically formed from one or more antigen binding arms of one or more antibodies, for recognising one or more analytes, and one or more copies of a metal-binding protein attached to the antigen binding arm Typically the antibody fragment used comprises the variable regions of the heavy and light chains, V_{H} and V_{L} joined by a flexible linker to create a single chained peptide (sc), usually termed scFv. When both moieties in the label are formed from protein and/or polypeptides (i.e. the label comprises a chimaeric protein) the label may be formed using recombinant techniques that are well known in the art. An illustration of this is provided in Figure 1. However, should any of the moieties be formed from other species, the labels may be made by simple attachment of one species to another.

Thus, the present invention also provides a method for forming a label for an analyte as defined above, which method comprises joining together a recognition moiety for attaching the label to the analyte and a moiety for binding the magnetic or magnetisable substance.

Further provided by the present invention is a method of processing a sample, which method comprises:
(a) contacting the sample with a label for an analyte, which label is attached to a magnetic or magnetisable substance, the label comprising:
   - a recognition moiety for attaching the label to the analyte; and
   - a moiety for binding or encapsulating the magnetic or magnetisable substance;
(b) subjecting the label to a magnetic field to influence the label;
(c) optionally analysing the label and/or the analyte to obtain information on an analyte that may be attached to the label.
wherein the moiety for binding or encapsulating the magnetic or magnetisable substance comprises a metal-binding protein, polypeptide, or peptide.

In one preferred example of the above method, the magnetic field may be employed to separate, purify and/or isolate the label, and/or any analyte that may be attached to the label, from one or more further substances in the sample. In this case the analysis step is not essential, because the objective of purification may be achieved without analysis. In other preferred methods, the analysis step is carried out, and typically comprises detecting the presence, absence, identity and/or quantity of an analyte attached to the label.

The present invention also provides a use of a label as defined above, in a nucleic acid, oligonucleotide, protein, polypeptide, infectious agent (e.g. a virus, virus particle or virus component) or cell purification method. The labels are preferably employed in sandwich assays, such as those carried out in a microfluidic device and/or a biosensor.

Still further provided by the present invention is a use of a moiety for binding a magnetic or magnetisable substance, wherein the moiety comprises a metal-binding protein, polypeptide, or peptide, and wherein the use is carried out using a microfluidic or a nanofluidic device.

The various moieties, including metal binding proteins, antibodies, and fusion proteins employed in the present invention will now be described in more detail.

Two metal-binding proteins were determined as good examples for further illustration of the present invention. These were ferritin and metallothionein II (MT2). It is preferred that fusion proteins are formed with either of these metal binding proteins, which comprise the variable domains of a murine antibody expressed as a single chain Fv (scFv) genetically fused to either the ferritin or the metallothionein II to give a recombinant protein.

### Metal Binding Proteins

The number of metal binding proteins described in the literature is still increasing. Many proteins store iron (Fe) as an oxyhydroxide-ferric phosphate or as haem, therefore complicating magnetising methods. Proteins such as ferritin are able to store thousands of iron ions within a cage-like structure.

As the endogenous iron within ferritin is not paramagnetic, it typically needs to be removed and replaced with a paramagnetic form without damaging the protein. Other metal binding proteins such as metallothionein II (MT2) hold fewer ions of metal in a loose lattice arrangement, and it may be easier to remove and replace these than with ferritin.

### Ferritin

Ferritin is a large protein, 12-nm diameter, with a molecular weight of 480kDa. The protein consists of a large cavity (8nm diameter) which encases iron. The cavity is formed by the spontaneous assembly of 24 ferritin polypeptides folded into four-helix bundles held by non-covalent bonds. Iron and oxygen form insoluble rust and soluble radicals under physiological conditions. The solubility of the iron ion is 10⁻¹⁸M. Ferritin is able to store iron ions within cells at a concentration of 10⁻⁴M.

The amino acid sequence, and therefore the secondary and tertiary structures of ferritin are conserved between animals and plants. The sequence varies from that found in bacteria; however, the structure of the protein in bacteria does not. Ferritin has an essential role for survival as studies using gene deletion mutant mice resulted in embryonic death. Ferritin has also been discovered in anaerobic bacteria.

Ferritin is a large multifunctional protein with eight Fe transport pores, 12 mineral nucleation sites and up to 24 oxidase sites that produce mineral precursors from ferrous iron and oxygen. Two types of subunits (heavy chain (H) and light chain (L)) form ferritin in vertebrates, each with catalytically active (H) or inactive (L) oxidase sites. The ratio of heavy and light chains varies according to requirements. Up to 4000 iron atoms can be localised in the centre of the ferritin protein.

The iron stored within ferritin is usually in the form of hydrated iron oxide ferrihydrite (5Fe₂O₃·9H₂O). It is possible to replace the ferrihydrite core with ferrimagnetic iron oxide, magnetite (Fe₃O₄). This may be achieved by removing the iron using thioglycolic acid to produce apoferritin. Fe(II) solution is then gradually added under argon or other inert gas with slow, controlled oxidation by the introduction of air, or an alternative oxidising agent.

### Metallothionein II

Metallothioneins are intracellular, low molecular weight, cysteine-rich proteins. These proteins are found in all eukaryotes and have potent metal-binding and redox capabilities. MT-1 and MT-2 are rapidly induced in the liver by a variety of metals, drugs and inflammatory mediators. The functions of MT-2 include zinc (Zn) homeostasis, protection from heavy metals (especially cadmium) and oxidant damage and metabolic regulation.

MT2 binds seven divalent transition metals via two metal binding clusters at the carboxyl (α-domain) and amino (β-domain) terminals. Twenty cysteine residues are involved in the binding process.

Chang *et al* describe a method of replacing the seven zinc (Zn²⁺) ions with manganese (Mn²⁺) and cadmium (Cd²⁺) ions. The resultant protein was shown to exhibit a magnetic hysteresis loop at room temperature. This could potentially mean that the protein is paramagnetic.

Toyama *et al* engineered human MT2 to construct an additional metal binding site. This could potentially increase the paramagnetic functioning of the MT2, and may be employed in the present invention.

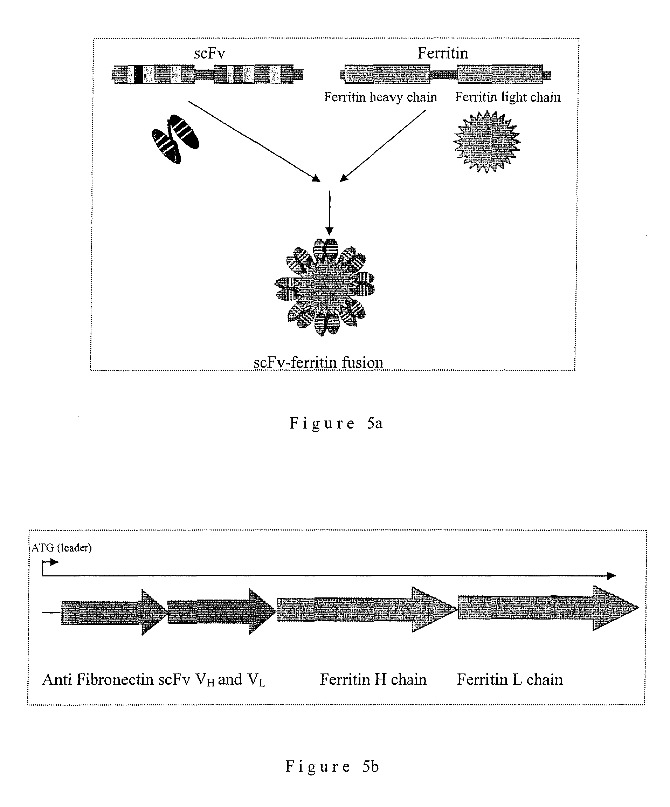

As ferritin and MT2 can potentially be magnetised, they provide an alternative to currently available magnetic beads. Using molecular biology techniques, the variable regions of antibodies can be linked to the genes coding for ferritin or MT2 to produce magnetic antibody like proteins (see Figures 5a and 5b). This may be demonstrated using an available scFv, such as anti-fibronectin scFv genes. Fibronectin is found in connective tissue, on cell surfaces, and in plasma and other body fluids. Over-expression of fibronectin genes has been found in a number of liver carcinomas and the protein has been shown to be implicated in wound healing; therefore diagnosis would have potential "theranostic" value. Thus, it is a preferred embodiment of the present invention is to employ anti-fibronectin scFv genes and ferritin heavy and light genes to generate a large, multi-valent fusion protein. The scFv may also be linked to the human MT2 gene to generate a smaller fusion protein.

The use of genetically fused recognition and paramagnetic domains on a single protein eliminates the need for chemical conjugations and the potential damage to the functional activity of proteins caused by chemical manipulation. The scFv or magnetisable domains may be replaced at will with relative ease.

The scFv may comprise anti-fibronectin heavy and light chains linked by a short chain of glycine and serine residues. It has been found that the V_{H}-linker-V_{L} constructs are robust and maintain binding, and therefore these are preferred.

Lee *et al* have found that genetically fusing the heavy chain of ferritin to the amino terminus of the light chain significantly increased the cytoplasmic solubility of recombinant ferritin in *E. coli.* This approach may also be used in the present invention. The scFv and ferritin genes are joined together via a short linker region composed of serine and four glycine residues for the same reasons as mentioned above for scFv fragments, i.e. as these residues are small, flexible and unlikely to interfere with other essential residues.

Ahn *et al* found that genes fused to the C-terminus of the heavy and light chains of ferritin were likely to be expressed within the ferritin molecule rather than on the surface. For this reason, the design of the scFv ferritin fusion construct preferably has the scFv at the N-terminal of the ferritin heavy chain.

### Antibodies

The following abbreviations will be used when discussing the antibodies that may be employed in the present invention.
- C_{H}: Antibody heavy chain constant domain
- C_{L}: Antibody light chain constant domain
- CDR: Complementarity determining region (of antibodies)
- Fab: Single antibody recognition fragment (after papain cleavage) consisting of the variable domains and the light constant chain and C_{H}1.
- F(ab)₂: Antibody recognition fragment (after papain cleavage)
- Fc: Crystallisable fragment (after papain cleavage) of antibodies (usually the CH₂ and CH₃ domains).
- Fr: Framework regions (of the variable part of antibodies)
- Fv: Variable fragment (of antibodies)
- Ig: Immunoglobulin
- MT: Metallothionein
- MT1: Metallothionein I
- MT2: Metallothionein II
- scFv: single chain variable fragment
- V_{H}: Antibody variable heavy domain
- V_{L}: Antibody variable light domain

The invention provides a magnetic antibody-like chimæric protein. The magnetic segment of the protein is composed of one or more copies of iron binding proteins, as described above. The recognition arm of the protein is composed of antibody fragments or receptors which bind the antigen of interest, which will be discussed in more detail below. The source of the antibodies is not especially limited, and antibodies may be derived from any species, or from phage display libraries, or from other recombinant systems.

A typical antibody portion of the protein of the invention is composed of the antigen binding sites of a murine monoclonal IgG1 antibody which binds fibronectin (known hereafter as the anti fibronectin scFv domain).

Antibodies are immunoglobulin proteins involved in the specific adaptive immune response. Each immunoglobulin has two distinct roles. One role is to bind antigen and the other is to mediate immune (effector) function. These effector functions include binding of the immunoglobulin to host tissues, immune cells and other immune proteins. Antibodies consist of four polypeptide chains (Figure 4). Two identical longer chains (known as the heavy chains) are covalently linked by disulfide bridges to each other at a region known as the hinge. Each heavy chain is also covalently linked via a disulfide bridge to identical shorter chains (known as light chains). Each polypeptide chain contains several domains (labelled V_{L} and C_{L} for the light chain and V_{H}, C_{H}1, C_{H}2 and C_{H}3 in Figure 4) which are each encoded for by exons within a gene. Each domain has a molecular weight of approximately 12.5kDa. There are five major antibody classes in humans; namely IgG, IgA, IgM, IgD and IgE and these may also have subclasses. Each antibody class has a characteristic effector region and therefore modulates the immune system in a different way. The antigen binding domain is located at the amino end of the immunoglobulin at regions known as the variable heavy (V_{H}) and variable light (V_{L}) domains. The effector domains are in the remainder of the antibody (constant regions).

Vertebrate immune systems are able to recognise and bind to millions of antigens. This is due in part to the remarkable antigenic diversity of antibodies. The variable domains of antibodies are encoded for by sets of genes which can be shuffled to generate variability. In addition, further modifications of the genes occur which is known as somatic mutation. The areas of antibody which are in direct contact with antigen (the recognition sequences) are the most variable regions. These regions are known as complementarity determining regions (CDRs). There are three of these regions on each polypeptide chain and these are represented as lighter lines in Figure 4b. Although the amino acid residues between the CDRs do not directly contact the antigen, they are of paramount importance in the forming the correct structure of the antigen binding region. For this reason, they are known as the framework regions.

Antibodies are produced in a specialist cell known as the B-cell. B-cells have an antibody on their surface which is able to bind a specific antigen. In other words, a single B-cell is able to "recognise" a single antigen via its surface antibodies. When this membrane bound antibody encounters an antigen, the B-cell undergoes maturation which ultimately leads to division and proliferation of the cell. The daughter cells from the original cell (or clone as it is known) are able to produce soluble (non-membrane bound) forms of antibody of the same specificity as the original membrane bound antibody. All antibodies produced from these daughter cells are known as monoclonal antibodies as the cells are derived from a single clone.

Antibodies for use *in vitro* have been produced for many years. Originally, polyclonal serum from immunised animals was the easiest method of obtaining antibodies. In 1975, pioneering work by Georges J.F. Köhler and César Milstein at Cambridge University led to the development of laboratory produced monoclonal antibodies. Their work involved fusing a B-cell from a mouse spleen with a myeloma cell to create an immortalised B-cell line known as a hybridoma.

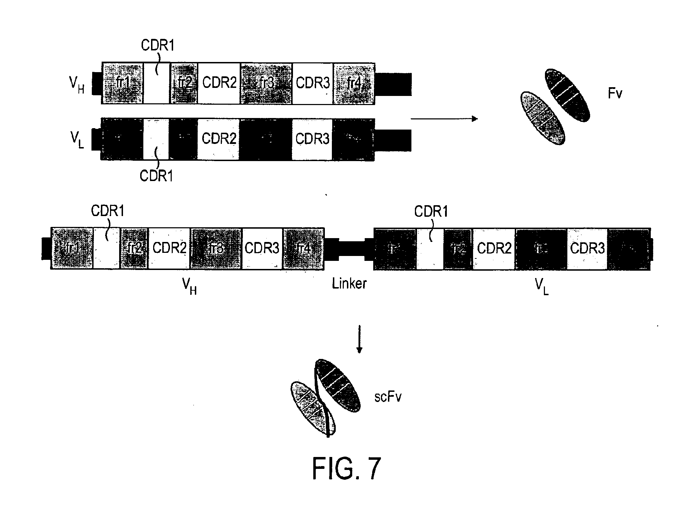

The antigen binding portions of antibodies can be used in isolation without the constant regions. This may be of some use in, for example, designing antibody like molecules better adapted at penetrating solid tumours. The V_{H} and V_{L} domains can be expressed in cells as an Fv fragment. Alternatively, the two domains can be linked by a short chain of small amino acids to form a single polypeptide known as a single chain Fv fragment (scFv), which has a molecular weight of approximately 25kDa (see Figure 7). The linker is composed of a number of small amino acids such as serine and glycine which do not interfere with the binding and scaffold regions of the scFv.

### Fusion protein design

In the present invention, the fusion proteins may be designed using the variable regions from an anti-fibronectin murine monoclonal IgG1 antibody to generate a scFv domain. The heavy and light chains of ferritin or the MT2 gene can be used to generate the magnetic domain of the antibody. The genes for the variable domains of the anti-fibronectin antibody are commercially available, and these are typically cloned into a plasmid vector to be expressed as a scFv. The scFv may be translated in the following order:
ATG start codon: leader sequence (for expression): heavy chain: glycine
serine linker: light chain.

### Plasmid generation

The genes for the human heavy and light chains of ferritin or human MT2 may be obtained from a human library, cloned using appropriately designed primers and inserted into the anti-fibronectin scFv plasmid vector at the 3' end of the antibody light chain with a terminal stop codon. Genes fused to the 3' end of the heavy and light chains of ferritin may be expressed within the ferritin molecule rather than on the surface. Therefore, the scFv ferritin fusion construct has the scFv at the N-terminal (corresponding to the 5' end) of the ferritin heavy chain. The scFv and ferritin or MT2 fusion proteins typically have a histidine tag (consisting of six histidine residues) at the C-terminus of the protein before the stop codon. This allows for the detection of the proteins in applications such as Western blotting, and for possible purification using metal affinity columns (such as nickel columns) or other tags (e.g. GST, b-galactosidase, HA, GFP) if the metal binding functions interfere. The sequences of the genes may be checked after plasmid production to ensure no mutations had been introduced.

Figure 5b is a diagrammatic representation of an exemplary ferritin fusion protein. The scFv heavy and light chains are represented by first two arrows respectively.

The sequence employed was SEQ ID 1 set out below:

SEQ ID 1













The scFv heavy and light chains are represented by italics in the amino acid sequence, heavy chain underlined. The bold text in the amino acid sequence represents the CDR regions of the variable domains. The two glycine/serine linkers are indicated in lower case, the second of which runs into the sequences of the heavy and light chains of ferritin in plain text, again heavy chain sequence underlined.

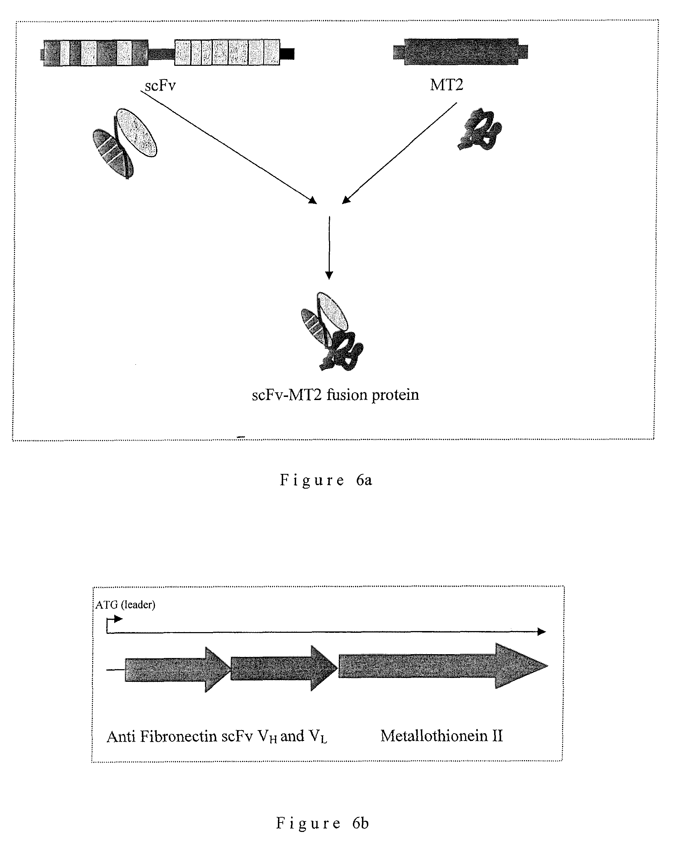

Figure 6b is a diagrammatic representation of an exemplary MT2 fusion protein. The sequence is represented by SEQ ID 2 below:

SEQ ID 2
LVQPGGSLRLSCAASGFTFSSFSMSWVRQAPGKGLEWVSSISGSSGTTYYADSVKGRFTI
SRDNSKNTLYLQMNSLRAEDTAVYYCAKPFPYFDYWGQGTLVTVSSGDgssggsggASTGEI
VLTQSPGTLSLSPGERATLSCRASQSVSSSFLAWYQQKPGQAPRLLIYYASSRATGIPDRFS
GSGSGTDFTLTISRLEPEDFAVYYCQQTGRIPPTFGQGTKVEIKsgggMDPNCSCAAGDS
CTCAGSCKCKECKCTSCKKSCCSCCPVGCAKCAQGCICKGASDKCSCCAPGSAGGS
GGDSMAEVQLLE.

The scFv sequence is in italics, with heavy chain underlined, bold text highlights CDRs. The two linker sequences are in lower case, with the second running into the metallothionein sequence given in normal text.

The scFv-ferritin and scFv-MT2 fusion proteins may be expressed in strains of *E*. *coli.* This is typically achieved by transforming susceptible *E*. *coli* cells with a plasmid encoding one or other of the fusion proteins. The expression plasmids typically contain elements for bacterial translation and expression as well as enhancer sequences for increased expression.

The plasmid also preferably contains a sequence for antibiotic resistance. When the bacterial cells are spread onto an agar nutrient plate containing the antibiotic, cells that do not contain the plasmid will not divide. Those cells that do contain the plasmid are able to grow in discreet colonies. Each cell in the colony is descended from a single cell or 'clone' (therefore the process is known as cloning).

The clones may be picked from the plate and grown in liquid media containing antibiotic. Fusion protein expression is generally initiated by the addition of an inducer (such as isopropyl β-D-1-thiogalactopyranoside or IPTG). The cells may be incubated for a limited amount of time before being harvested. The cells may be lysed using urea, and the lysates analysed, e.g. by SDS-PAGE and Western blotting.

### Protein detection and purification

The protein expression profile of clones may be assessed using SDS-PAGE (sodium dodecyl sulphate polyacrylamide gel electrophoresis) and Western blotting. In these assays, proteins are chemically denatured (by severing sulphur bonds using chemicals such as β-mercaptoethanol and/or by the addition of SDS which eliminates intra-bond electro-static charges). Cell lysates are added to a well at the top of the gel. An electric current (DC) is then applied to the gel and proteins migrated through the gel according to their size. The proteins are then visualised by staining the gel with a dye. Specific proteins are probed for by transferring the separated proteins onto a nitrocellulose membrane (again by using an electric current). Specific enzyme-linked antibodies are incubated on the sheet and substrate (a colourimetric, luminescent or fluorescent chemical) is added to visualise proteins.

The clone with the highest level of expression is usually expanded and grown at large scale (1 litre). The cells are induced as above and harvested.

The harvested cells are lysed and the proteins purified using, for example, metal affinity chromatography. Other methods of purification may be employed, if desired, including fibronectin affinity columns.

### Protein characterisation assays

### Size analysis

The proteins may be assayed for size using SDS-PAGE and Western blotting analysis and chromatography techniques.

### Surface Plasmon resonance

The binding of the fusion proteins to fibronectin may be assessed using surface plasmon resonance (SPR). SPR is a technique where the changes in the refractive index of light when a molecule binds to a thin metal film can be measured. Fibronectin is immobilised to the metal surface of a SPR chip and the fusion protein flowed over the chip's surface. When the fusion protein binds, the kinetics of binding (association, dissociation and affinity) may be assessed using SPR. Results obtained using a SPR are usually in the form of a sensorgram.

The binding of the fusion proteins may also be assessed by ELISA. The assays for determining binding involve coating microtitre plates with fibronectin or anti-ferritin antibodies. The uncoated sites on the plate are blocked using bovine serum albumin (BSA). The fusion protein is then incubated on the plates. The plates are then washed and incubated with anti-ferritin antibodies and washed again. Enzyme-linked antibodies are then incubated on the plates before the plates are washed prior to the addition of a substrate.

### Magnetisation of ferritin and scfv-ferritin

The iron within ferritin is not paramagnetic. The iron is usually in the form of Fe (III). In order to produce paramagnetic ferritin, the iron with ferritin (and ultimately, the fusion protein) is removed without damaging the protein; the iron was then replaced with a paramagnetic form (Fe (II)).

There are several forms of iron oxide and not all these forms are equally magnetic. E.g. FeO, Fe₂O₃ and Fe₃O₄. Iron oxide (Fe₃O₄) or ferrous ferric oxide, also known as magnetite or lodestone is the most magnetic form.

### Characterisation of the scfv-ferritin and scFv-MT2 fusion proteins

Physical characterisation of the treated proteins may be undertaken by a number of techniques, which typically include a combination of electron microscopy, diffraction (X-ray and/or electron) and Mossbauer spectroscopy.

The invention will now be described in more detail, by way of example only, with reference to the following specific embodiments.

### EXAMPLES

### EXAMPLE 1- Design and manufacture of fusion proteins

In order to exemplify the invention, fusion proteins were designed, using commercially available murine anti-fibronectin antibody. Fusion proteins consisting of anti-fibronectin scFv genetically linked by short flexible linkers to either MT2, or ferritin were produced. This Example details the construction of the fusion proteins, their characterisation and isolation.

The design of the anti-fibronectin ferritin or MT2 fusion proteins was based on cloning the V_{H} and V_{L} genes from a mouse anti-fibronectin antibody into a vector. Both genes were linked by short, flexible linkers composed of small non-charged amino acids. Immediately at the 3' end of the V_{L} gene, another short flexible linker led into either the ferritin genes or the MT2 gene. Both fusion proteins had a six-histidine region for purification on nickel columns. The fusion protein translation was terminated at a stop codon inserted at the 3' end of the ferritin light gene or the MT2 gene. The plasmid vector containing all these elements was used to transform bacteria for expression.

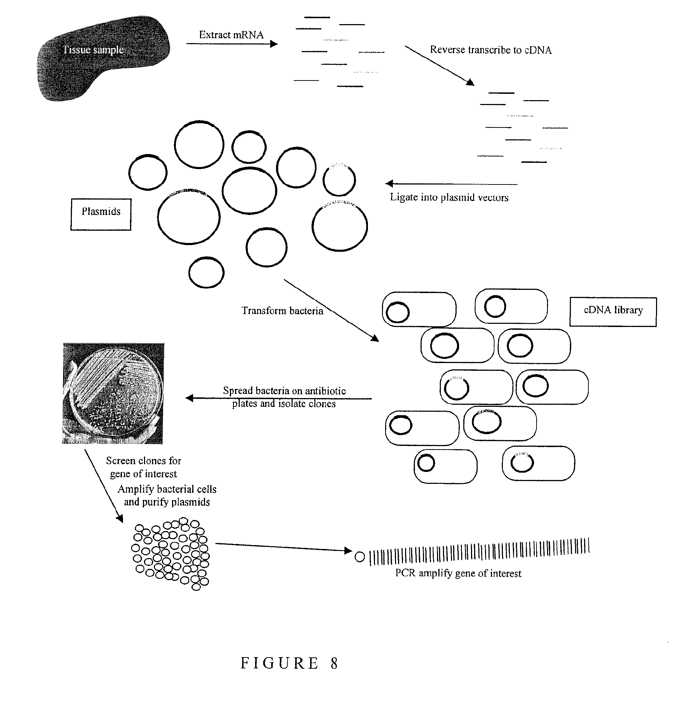

The genes for the ferritin and MT2 were obtained from cDNA libraries. A cDNA library is formed by obtaining mRNA from cells or tissues, reverse transcribing the RNA to cDNA using an enzyme known as reverse transcriptase and cloning each individual cDNA into a plasmid vector (see Figure 8).

### Generation of the anti-fibronectin: ferritin fusion protein

### Background

Ferritin is a 12-nm diameter protein with a molecular weight of approximately 480kDa. The protein consists of a large cavity (8nm diameter) which encases iron. The cavity is formed by the spontaneous assembly of 24 ferritin polypeptides folded into four-helix bundles held by non-covalent bonds. The amino acid sequence and therefore the secondary and tertiary structures of ferritin are conserved between animals and plants. The structure of the protein in bacteria is the same as eukaryotes, although the sequence is different. Two types of subunits (heavy chain (H) and light chain (L)) form ferritin in vertebrates, each with catalytically active (H) or inactive (L) oxidase sites. The ratio of heavy and light chains varies according to requirement. The amino acid sequences of the ferritin heavy and light chains used in the construction of the fusion proteins are:
Ferritin heavy chain (molecular weight 21096.5 Da): 
MTTASTSQVRQNYHQDSEAAINRQINLELYASYVYLSMSYYFDRDDVALKNFAKYF
LHQSHEEREHAKLMKLQNQRGGRIFLQDIKKPDCDDWESGLNAMECALHLEKNVN
QSLLELHKLATDKNDPHLCDFIETHYLNEQVKAIKELGDHVTNLRKMGAPESGLAEY
LFDKHTLGDSDNES
Ferritin light chain (molecular weight 20019.6 Da):

Together with the anti-fibronectin scFv amino acid sequences, the predicted sequence of a single polypeptide of the fusion protein is (with the linker sequences between the heavy and light antibody genes and between the antibody light chain and ferritin heavy chain highlighted in lower case):

LVQPGGSLRLSCAASGFTFSSFSMSWVRQAPGKGLEWVSSISGSSGTTYYADSVKGR
FTSRDNSKNTLYLQMNSLRAEDTAVYYCAKPFPYFDYWGQGTLVTVSSGDgssggsgg

ASTGEIVLTQSPGTLSLSPGERATLSCRASQSVSSSFLAWYQQKPGQAPRLLIYYASSR
ATGIPDRFSGSGSGTDFTLTISRLEPEDFAVYYCQQTGRIPPTFGQGTKVEIKsgggMTT
ASTSQVRQNYHQDSEAAINRQINLELYASYVYLSMSYYFDRDDVALKNFAKYFLHQ
SHEEREHAKLMKLQNQRGGRIFLQDIKKPDCDDWESGLNAMECALHLEKNVNQSLL
ELHKLATDKNDPHLCDFIETHYLNEQVKAIKELGDHVTNLRKMGAPESGLAEYLFD
KHTLGDSDNESMSSQIRQNYSTDVEAAVNSLVNLYLQASYTYLSLGFYFDRDDVAL
EGVSHFFRELAEEKREGYERLLKMQNQRGGRALFQDIKKPAEDEWGKTPDAMKAA
MALEKKLNQALLDLHALGSARTDPHLCDFLETHFLDEEVKLIKKMGDHLTNLHRLG
GPEAGLGEYLFERLTLKHD

The molecular weight of the polypeptide component was 65.550kDa.

### Assembly of the anti-fibronectin: ferritin fusion protein genes

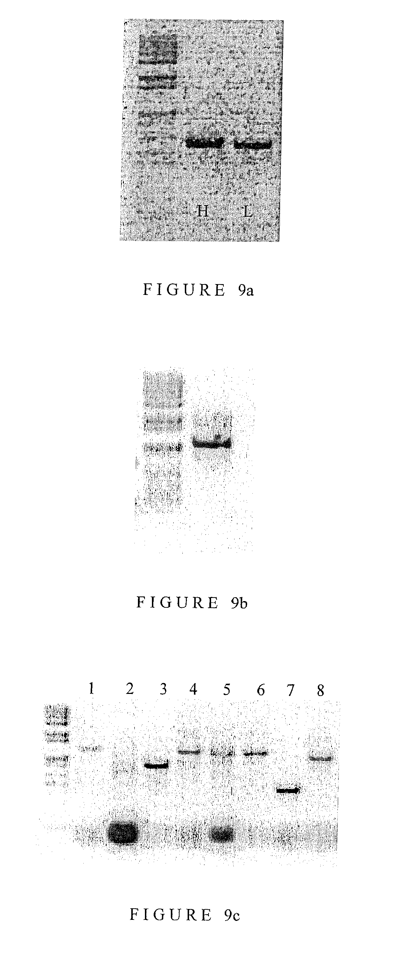

Ferritin heavy and light chain genes were amplified from a human liver cDNA library using PCR (see Figure 9a). The PCR products were of the expected size (∼540bp). These PCR products were ligated using overlapping PCR (Figure 9b - the product is of the expected size).

The overlap PCR product was gel purified and ligated into a sequencing vector for sequencing analysis. This involved transforming bacteria with the sequencing vector containing the ferritin heavy and light chain overlapped genes. The transformed bacteria were then spread on an antibiotic containing plate to separate clones. The cells were incubated overnight to allow colonies to form. Individual colonies were then picked from the plate and grown in liquid media. The plasmids from each clone were isolated and analysed using PCR (Figure 9c). Clone 4 was found to contain the expected sequence. The DNA from this clone was therefore subsequently used in all further work.

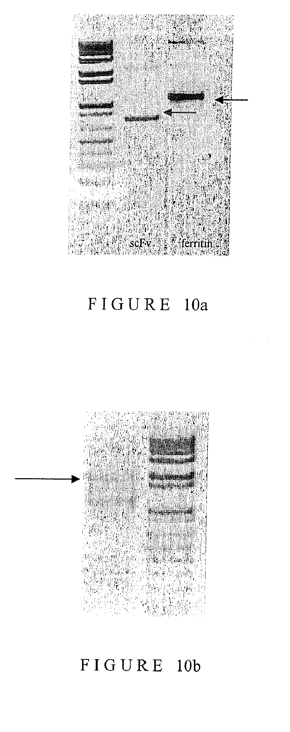

The variable heavy and light chain genes for a murine anti human fibronectin antibody were PCR amplified from a monoclonal hybridoma. These genes have previously been joined by a flexible linker region to form a scFv. This scFv gene fusion was amplified using PCR. The DNA gel of this amplification can be seen in Figure 10a alongside the ferritin polygene overlap product. The relevant bands were excised from the gel and the DNA purified. This was then used in a further overlap PCR to conjugate the scFv and ferritin polygene (Figure 10b). The arrowed band is of the expected size for the scFv:ferritin fusion. This was excised and the DNA purified for further use.

The primers used to do this contained sequences to allow for endonuclease (enzymes able to cut specific sequences of double stranded DNA) restriction of the DNA for ligation into a plasmid.

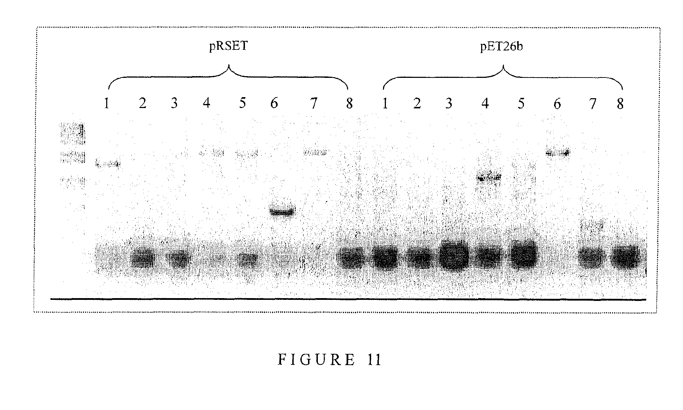

After gel purification, the scFv:ferritin PCR product was restricted using the restriction enzymes (endonucleases) *Bam* H1 and *Eco*R1. The purified restricted products were subsequently cloned into two expression vectors; pRSET and pET26b. Clones were isolated as before and the results of a PCR to identify positive clones can be seen in Figure 11.

Colonies 3-5 and 7 from the set containing the plasmid pRSET and colony 6 from the set containing the plasmid pET26b were selected for sequence analysis.

The resulting data demonstrated that clones pRSET 4 and 5 and pET26b clone 6 contained the scFv:ferritin construct. The clone pRSET 4 was used for protein expression.

### Anti-fibronectin scFv:ferritin fusion protein expression

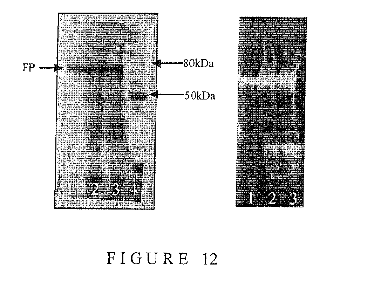

To validate the expression of the fusion protein, three 5ml cultures were grown in LB broth (Luria-Bertani broth: 10g tryptone, 5g yeast extract, 10g NaCl per litre). The cells were induced to express protein using IPTG (isopropyl β-D-1-thiogalactopyranoside) at varying times. The cultures were then lysed in 8M urea and analysed using SDS-PAGE. The gels were stained using Coomassie blue for protein content (results in Figure 12). Western blots using an anti-polyhistidine antibody were performed to specifically identify the fusion protein (Figure 12).

The time-points for induction were 2, 3 and 4 hours after inoculation.

The bands seen in the blot demonstrated that the fusion protein was being expressed and could be detected using an anti-histidine antibody. The polypeptide was approximately 75-85kDa in size. The expression yields were relatively high and over-expression was evident as the fusion protein bands correspond to the very dark bands seen in the Coomassie blue stained gel. Inducing 3 hours after inoculation gave relatively high levels of expression and was used for subsequent expression.

### Generation of the anti-fibronectin:MT2 fusion protein

### Background

Metallothioneins are intracellular, low molecular weight, cysteine-rich proteins. These proteins are found in all eukaryotes and have potent metal-binding and redox capabilities. MT-1 and MT-2 are rapidly induced in the liver by a variety of metals, drugs and inflammatory mediators. MT2 binds seven divalent transition metals via two metal binding clusters at the carboxyl (α-domain) and amino (β-domain) terminals. Twenty cysteine residues are involved in the binding process.

The sequence of MT2 is:

MDPNCSCAAGDSCTCAGSCKCKECKCTSCKKSCCSCCPVGCAKCAQGCICKGASDK
CSCCAPGSAGGSGGDSMAEVQLLE

Together with the anti-fibronectin scFv amino acid sequences, the predicted sequence of a single polypeptide of the fusion protein is (with the linker sequences between the heavy and light antibody genes and between the antibody light chain and MT2 heavy chain highlighted in lower case):

LVQPGGSLRLSCAASGFTFSSFSMSWVRQAPGKGLEWVSSISGSSGTTYYADSVKGR
FTISRDNSKNTLYLQMNSLRAEDTAVYYCAKPFPYFDYWGQGTLVTVSSGDgssggsgg
ASTGEIVLTQSPGTLSLSPGERATLSCRASQSVSSSFLAWYQQKPGQAPRLLIYYASSR
ATGIPDRFSGSGSGTDFTLTISRLEPEDFAVYYCQQTGRIPPTFGQGTKVEIKsgggMDP
NCSCAAGDSCTCAGSCKCKECKCTSCKKSCCSCCPVGCAKCAQGCICKGASDKCSC
CAPGSAGGSGGDSMAEVQLLE

### Assembly of the anti-fibronectin:MT2 fusion protein genes

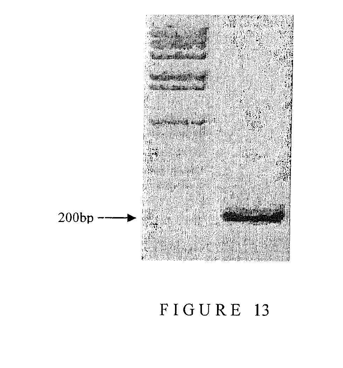

The metallothionein II genes were amplified from a human liver cDNA library using PCR (Figure 13). The PCR products were of the expected size (∼200bp).

The PCR product was restricted using the *Bgl* II restriction enzyme and ligated into a previously cut plasmid (Factor Xa vector).

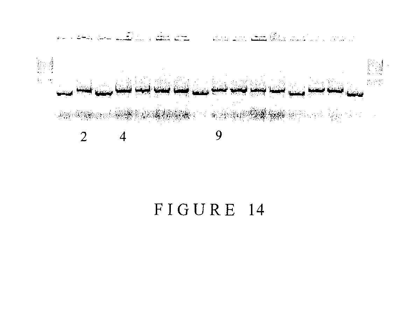

Colony PCR of selected clones revealed bands for all clones selected (Figure 14). Clones 2, 4 and 9 were selected for sequencing analysis. Clone 9 was used in further work.

### Anti-fibronectin scFv:MT2 fusion protein expression

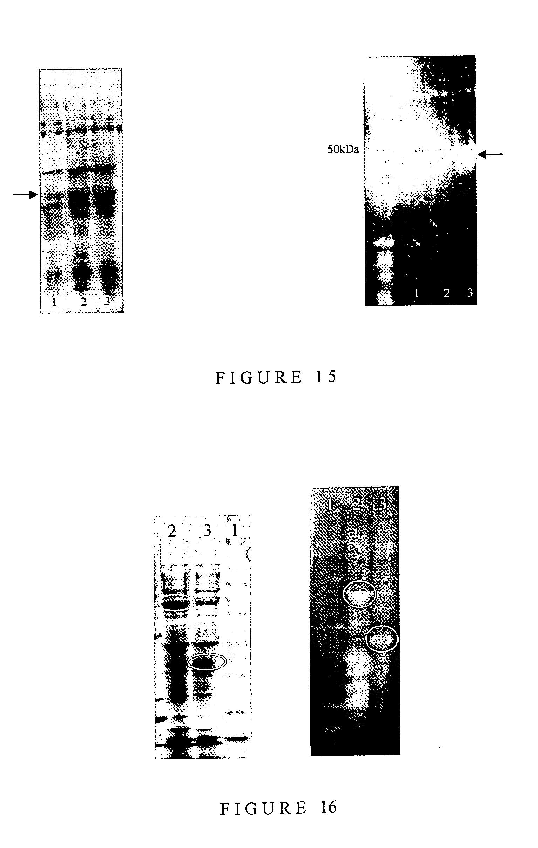

To validate expression of the scFv:MT2 fusion protein, three 5ml cultures were grown in LB broth induced (IPTG) at different time-points as with the ferritin fusion protein. The cultures were lysed in 8M urea and analysed using SDS-PAGE gels stained with Coomassie blue and blotted using an anti-histidine antibody (Figure 15). Cells induced 4 hours after inoculation produced slightly more protein (lane 3 on both gels). These growth conditions were used for subsequent protein expression.

### Purification of fusion proteins

The isolation of soluble protein by isolating, washing and re-solubilising inclusion bodies was employed.

The protocol takes approximately one week to complete. Photographs of a Coomassie blue stained gel and western blot of the re-solubilised scFv:ferritin and scFv:MT2 fusion proteins can be seen in Figure 16. The fusion proteins are circled - ferritin is in lane 2 on both gels and MT2 is in lane 3 of both gels. A protein molecular weight ladder is in lane 1.

From this, it can be seen that the fusion proteins were successfully expressed and concentrated. These proteins were be used in magnetising protocols and further experiments.

### EXAMPLE 2 - SPR analysis

Anti-fibronectin ferritin and MT2 fusion protein inclusion body preparations were used in surface plasmon resonance (SPR) assays using a SensiQ instrument (ICX Nomadics).

For these experiments, a fibronectin peptide was coupled to the surface of a carboxyl chip. The fusion protein preps were then flowed over the chip and association (Kₐ) and dissociation kinetics (K_{d}) determined.

### Fusion protein samples for analysis

Six samples of each fusion protein, with varying concentration from 0.0013 - 0.133µM were produced in running buffer as set out in Table 2 and Table 3 below.

**Table 2 - Metallothionein Fusion Protein 75kDa: 40µl 100µg/ml 75kDa / 360µl running buffer to give 400µl 10µg/ml (0.133µM) then:**

| µM FP | µg/ml FP | µl of 10µg/ml FP | µl of running buffer |
|---|---|---|---|
| 0.0013 | 0.1 | 20 (of µg/ml) | 180 |
| 0.0065 | 0.5 | 10 | 190 |
| 0.013 | 1 | 20 | 180 |
| 0.05 | 3.75 | 75 | 125 |
| 0.1 | 7.5 | 150 | 50 |
| 0.133 | 10 | 400 | 0 |

**Table 3 - Ferritin ED-B Fusion Protein 270kDa: 144µl 100µg/ml 270kDa / 256µl running buffer to give 400µl 36µg/ml (0.133µM) then:**

| µMFP | µg/ml FP | µl of 36µg/ml FP | µl of running buffer |
|---|---|---|---|
| 0.0013 | 0.36 | 20 (of 3.6µg/ml) | 180 |
| 0.0065 | 1.8 | 10 | 190 |
| 0.013 | 3.6 | 20 | 180 |
| 0.05 | 9 | 75 | 125 |
| 0.1 | 18 | 150 | 50 |
| 0.133 | 36 | 400 | 0 |

### Metallothionein

Sample (Cycles 1-6) = 20µl 0.0013 - 0.133 µM Metallothionein Fusion Protein

Assay run = MAb & Gly assay cycle (as above)

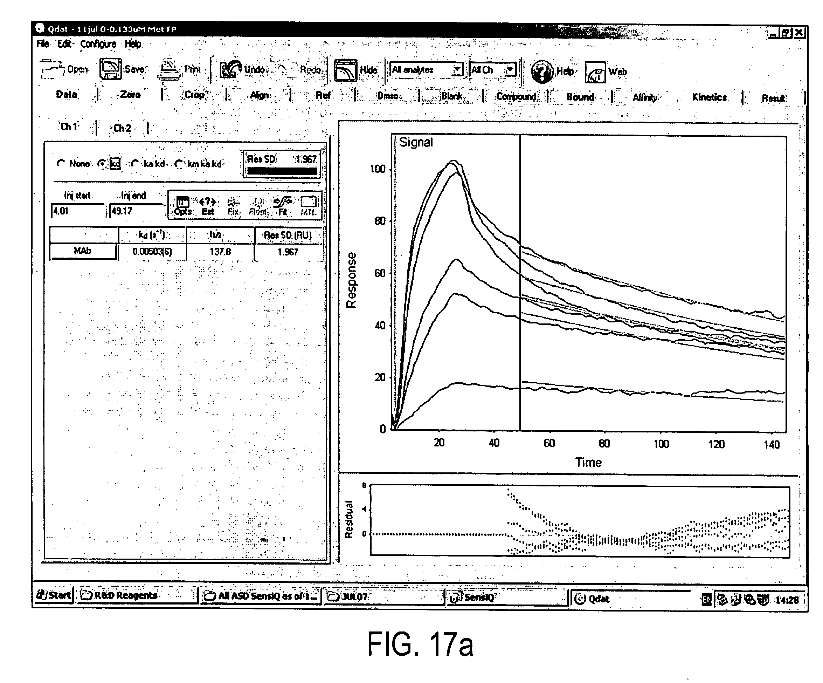

Sensograms from the above cycles were overlaid using the SensiQ Qdat analysis software, and a model fitted to the data to calculate kinetic parameters (Kₐ, K_{d}). The best estimate of the K_{d} was achieved by fitting a model to just the dissociation part of the data. The result is shown in Figure 17a. This relates to a K_{d} of 0.00503 s⁻¹ to give a K_{d} of 2.289 x 10⁻⁹ M (Kₐ 2.197 x 10⁶ M⁻¹s⁻¹)

### Ferritin

Sample (Cycles 1-6) = 20µl 0.0013 - 0.133 µM Ferritin Fusion Protein

Assay run = MAb & Gly assay cycle (as above)

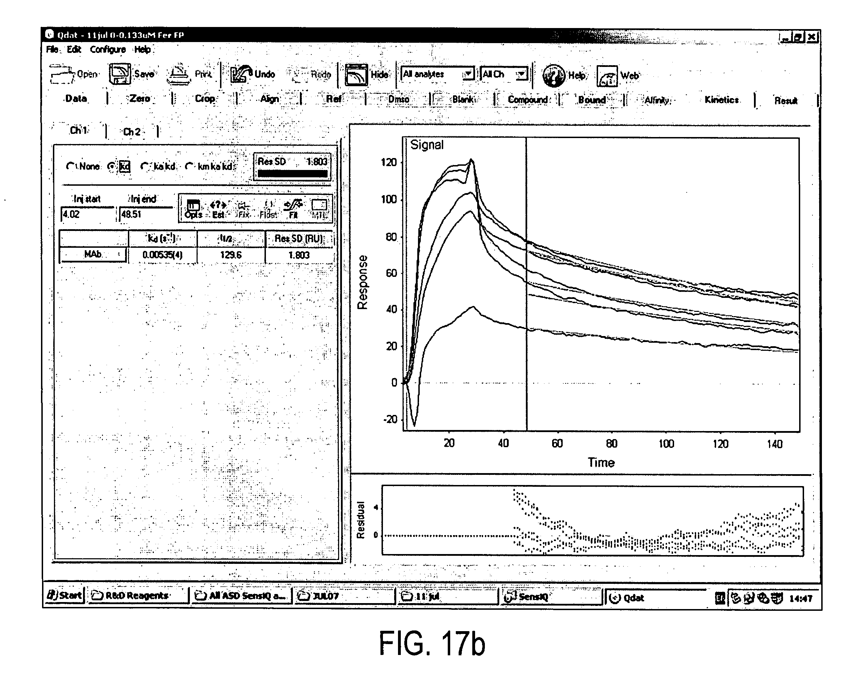

Sensograms from the above cycles were overlaid using the SensiQ Qdat analysis software and a model fitted to the data to calculate kinetic parameters (Kₐ, K_{d}). The best estimate of the K_{d} was achieved by fitting a model to just the dissociation part of the data. The result is shown in Figure 17b. This relates to a K_{d} of 0.00535 s⁻¹ to give a K_{d} of 6.538 x 10⁻¹⁰ M (Kₐ 8.183 x 10⁶ M⁻¹s⁻¹)_{.}

### Results

From the above experimental data, it was determined that fibronectin extra domain B (aa 16-42) antigen was successfully coated onto the SensiQ chip As expected, both the 75kDa Metallothionein Fusion Protein and the 270kDa Ferritin Fusion Protein recognised and bound to the antigen in a specific manner. Kinetic data on the interactions of the fusion proteins with the antigen were estimated and were found to be similar and in the expected range for both fusion proteins i.e. K_{d}s in the 10⁻⁹ M range compared to 10⁻⁸ M to 10⁻¹⁰ M for most antibody / antigen interactions.

Thus, the values obtained using this instrument suggest binding affinities which compare favourably with the binding affinities of relatively high affinity antibodies. In addition, the data obtained suggest that the fusion proteins have multiple binding sites for antigen. This was expected for the ferritin fusion protein. However, this was not expected for the MT2 fusion protein and would suggest that the fusion protein is forming dimers or higher order multimeric proteins which would increase the avidity of binding.

### EXAMPLE 3 - Magnetising ferritin

Ferritin normally contains hydrated iron (III) oxide. In order to produce paramagnetic ferritin, these ions were replaced with magnetite (Fe₃O₄) which has stronger magnetic properties. The method used for this experiment involved the addition to apoferritin of iron ions and oxidation of these ions under controlled conditions.

### Materials

- Reverse osmosis water (RO water)
- 50mM N-(1,1-Dimethyl-2-hydroxyethyl)-3-amino-2-hydroxypropanesulfonic acid (AMPSO) buffer pH8.6 (Sigma A6659)
- 0.1M Sodium acetate buffer pH4.5
- Phosphate buffered saline (PBS) 10mM phosphate, 140mM NaCl pH 7.4
- Trimethylamine-N-oxide (TMA) (Sigma 317594)
- 0.1M ammonium iron (II) sulphate
- Horse spleen apoferritin (Sigma A3641)

### Method

Trimethylamine-N-oxide (TMA) was heated in an oven to 80°C for 30 minutes to remove Me₃N before cooling to room temperature. 114mg TMA was added to 15ml RO water to produce a 0.07M solution. The iron and TMA solutions were purged with N₂ for 15 minutes before use.

AMPSO buffer (1 litre) was de-aerated with N₂ for an hour. 3.0ml apoferritin (66mg/ml) was added to the AMPSO buffer and the solution de-aerated for a further 30 minutes. The AMPSO/apoferritin solution in a 1 litre vessel was placed into a preheated 65°C water bath. The N₂ supply line was removed from within the solution and suspended above the surface of the solution to keep the solution under anaerobic conditions. The initial addition of iron ammonium sulphate scavenges any residual oxygen ions that may be in the solution.

Aliquots of the 0.1M iron ammonium sulphate and TMA buffers were added every 15 minutes as follows:

| | |
|---|---|
| 1^{st} addition | 600µl 0.1M iron ammonium sulphate |
| 2^{nd} addition | 600µl 0.1M iron ammonium sulphate and 400µl TMA |
| 3^{rd} addition | 600µl 0.1M iron ammonium sulphate and 400µl TMA |
| 4^{th} addition | 600µl 0.1M iron ammonium sulphate and 400µl TMA |
| 5^{th} addition | 900µl 0.1M iron ammonium sulphate and 600µl TMA |
| 6^{th} addition | 900µl 0.1M iron ammonium sulphate and 600µl TMA |
| 7^{th} addition | 900µl 0.1M iron ammonium sulphate and 600µl TMA |
| 8^{th} addition | 900µl 0.1M iron ammonium sulphate and 600µl TMA |

Upon the latter additions of Fe and TMA, the solution colour changed from a straw colour to dark brown with dark particulates dispersed throughout. This solution is termed "magnetoferritin" from this point onwards.

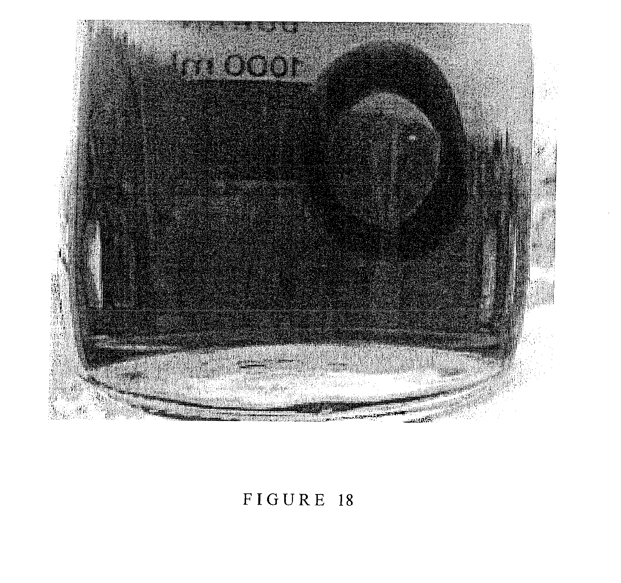

The magnetoferritin solution was incubated at room temperature overnight with a strong neodymium ring magnet held against the bottle. The following day, dark solid material had been drawn towards the magnet as can be seen in the photographs in Figure 18.

### Concentration of magnetoferritin

Five hundred millilitres of the magnetoferritin solution was passed through 5 Macs® LS columns on magnets (with approximately 100ml magnetoferritin passing through each column). The solution which flowed through the columns (termed 'flow-through') was collected in Duran bottles. The captured material from each column was eluted using 3ml PBS by removing the columns from the magnets, adding the 3mls PBS and using the supplied plunger resulting in approximately 4.5ml from each column. Approximately 1ml was stored at 2-8°C for later analysis (termed 'pre-dialysis concentrated magnetoferritin'). The remainder of the eluted solution (∼20ml) was dialysed (termed 'post-dialysis concentrated magnetoferritin') against 5 litres of PBS at 4°C overnight to remove excess Fe and TMA. The change in colour of the solution was noted. The original magnetoferritin was dark brown, the flow through straw coloured and the Macs® column concentrated material dark brown to black.

Dialysis tubing (Medicell International Ltd. Molecular weight cut-off 12-14000 Daltons ∼15cm) was incubated in RO water for ten minutes to soften the tubing. The magnetically isolated concentrated magnetoferritin was transferred to the dialysis tube and incubated in 5 litres PBS at 2-8°C with stirring overnight. The PBS solution was refreshed three times the following day at two hour intervals with dialysis continuing at 2-8°C.

### Analysis of magnetoferritin

In order to compare the amount of magnetic protein isolated using the magnet, enzyme linked immunosorbant assay (ELISA) analysis was performed.

### Materials

- Carbonate Buffer (0.159g sodium carbonate and 0.3g sodium bicarbonate in 100mls RO water).
- Phosphate buffered saline (PBS) 10mM phosphate, 140mM NaCl pH 7.4
- 1% bovine serum albumin (BSA (Celliance 82-045-2)) in PBS
- Horse spleen apoferritin (Sigma Aldrich A3641)
- Rabbit anti-horse ferritin antibody (Sigma Aldrich F6136)
- Goat anti-rabbit antibody (Sigma A3687)
- Substrate liquid stable phenolphthalein phosphate
- Stop Solution (212g sodium carbonate, 110.5g 3-(cyclohexylamino)-1-propanesulfonic acid (CAPS), 217g ethylenediamine tetraacetic acid (EDTA) 80g sodium hydroxide, water to 5 litres)
- Maxisorb Microtitre plate (NUNC Cat: 468667)

### Method

Dilutions of apoferritin were made (50µg/ml, 25µg/ml, 12.5µg/ml, 6.25µg/ml, 3.125µg/ml and 1.5625µg/ml) for quantification of the magnetoferritin.

The magnetoferritin (unpurified), concentrated pre-dialysis, post-dialysis and flow through was diluted in carbonate buffer at the following dilutions:
Magnetoferritin, pre-dialysis and post-dialysis dilutions:
   100, 200, 400, 800, 1600, 3200, 6400 and 12800 fold dilution.
Flow-through:
   10, 20, 40, 80, 160, 320, 640 and 1280 fold dilution.

100µl of each solution was added to wells of a microtitre plate in duplicate. Carbonate buffer (100µl) was added to two wells as a negative control. The plate was incubated overnight at 4°C. The next day, the solution was flicked off and the wells blocked using 200µl 1% BSA at room temperature for an hour. After washing three times with 300 µl PBS per well, the wells were patted dry before the addition of 100µl 10µg/ml anti-horse ferritin antibody. This was incubated for an hour at room temperature before being removed and wells washed as before. AP-conjugated anti rabbit antibody was diluted 1 in 3500 in PBS to give a concentration of 7.43µg/ml and incubated at room temperature for an hour. The antibody conjugate was removed and wells washed as before. AP substrate (100µl) was added to each well and allowed to develop for 15 minutes before the addition of stop solution. Absorbances were recorded using a Varioskan Flash instrument (Thermo Fisher).

The Macs® columns retained over 35 times the amount of magnetoferritin found in the flow through indicating that magnetisation of the protein had been successful.

*Production of apoferritin*/*demineralisation of horse spleen ferritin.*

### Materials

- 0.1M sodium acetate buffer pH 4.5
- Thioglycolic acid (Sigma T6750)
- Horse Spleen Ferritin (Sigma 96701)
- Phosphate buffered saline (PBS) 10mM phosphate, 140mM NaCl pH 7.4

### Method

Dialysis tubing was softened in RO water for 10 minutes. 10ml 0.1M sodium acetate buffer was added to 1ml Horse Spleen Ferritin (125mg/ml) in the dialysis tubing which was clipped at both ends. The dialysis bag was transferred to 0.1M sodium acetate buffer (∼800ml) which had been purged with N₂ for one hour. Thioglycolic acid (2ml) was added to the buffer and N₂ purging was continued for two hours. A further 1ml thioglycolic acid was added to the sodium acetate buffer followed by another thirty minutes of N₂ purging. The sodium acetate buffer (800ml) was refreshed and purging continued. The demineralisation procedure was repeated until the ferritin solution was colourless. The N₂ purge was stopped and the apoferritin solution was dialysed against PBS (2L) for 1h with stirring. The PBS was refreshed (3 litres) and the apoferritin solution was dialysed in PBS at 2-8°C overnight.

### Results

The ferritin solution changed colour during the procedure from light brown to colourless indicating removal of iron.

### Analysis of heat treatment on the anti-fibronectin.ferritin fusion protein

### Materials

- Carbonate buffer (0.159g sodium carbonate, 03g sodium bicarbonate in 100mls water)
- Phosphate buffered saline (PBS)
- 1% bovine serum albumin (BSA (Celliance 82-045-2)) in PBS
- Fibronectin peptide
- Anti-fibronectin:ferritin fusion protein (scFv:ferritin)
- Anti-human ferritin murine monoclonal antibody (Santa Cruz SC51887)
- Anti-mouse alkaline phosphatase antibody (Sigma A3 5 62)
- Substrate liquid stable phenolphthalein phosphate
- Stop Solution (212g sodium carbonate, 110.5g 3-(cyclohexylamino)-1-propanesulfonic acid (CAPS), 217g ethylenediamine tetraacetic acid (EDTA) 80g sodium hydroxide, water to 5 litres)
- Maxisorb Microtitre plate (NUNC Cat: 468667)

### Method

100µl (at 100µg/ml) scFv:ferritin was transferred to a thin walled PCR tube and heated in a thermocycler at 60°C for 30 minutes.

Wells of a microtitre plate were coated with fibronectin peptide (supplied at 1.5mg/ml) diluted in carbonate buffer to 15µg/ml and incubated overnight at 4°C. Excess solution was flicked off and the plate blocked using 1% BSA in PBS for 1 hour at room temperature. This was flicked off and the plate washed three times using PBS. The scfv:ferritin fusion protein and heat treated scFv:ferritin fusion protein were added to wells at a concentration of 33µg/ml (100µl each). The ferritin fusion proteins were incubated for 2 hours at room temperature before being removed and the wells washed as before. Mouse anti-ferritin antibody was added at a concentration of 20µg/ml and added at a volume of 100µl to each well and incubated at room temperature for an hour. This was removed and the wells washed as before. Goat anti-mouse AP conjugated antibody was diluted (50µl + 950µl PBS) and added at a volume of 100µl to all wells. This was incubated at room temperature for an hour and removed as before. Substrate was added to all wells and incubated at room temperature for 45 minutes and the reaction stopped using stop buffer. Absorbances were recorded using a Varioskan Flash instrument (Thermo Fisher Electron).

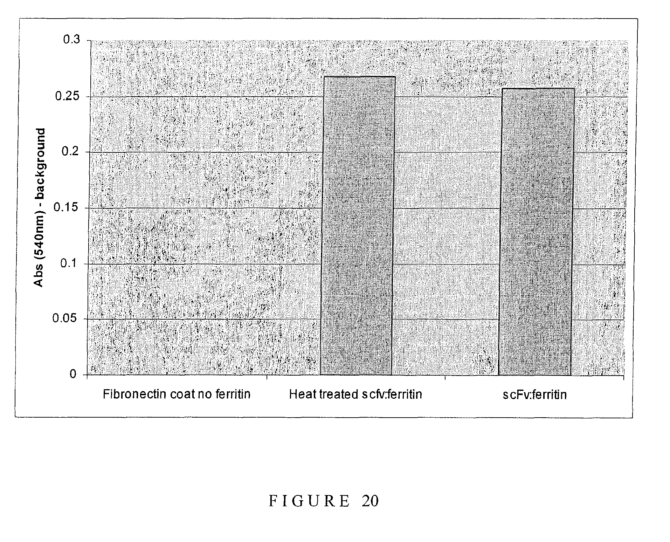

The scFv:ferritin retains binding ability to fibronectin and remains detectable by the anti-human ferritin monoclonal antibody after heating to 60°C for 30 minutes (Figure 20).

### Anti fibronectin:ferritin fusion protein demineralisation

### Materials

- Anti-fibronectin:ferritin fusion protein (scFv:ferritin).
- 0.1M sodium acetate buffer
- Thioglycolic acid (70% w/w Sigma T6750)
- Phosphate buffered saline (PBS) 10mM phosphate, 140mM NaCl pH 7.4

### Method

The scFv:ferritin fusion protein was thawed from -20°C to room temperature. Nine millilitres of 100µg/ml was dispensed into softened dialysis tubing. The tubes which had contained the fusion protein were rinsed with a total of 1ml sodium acetate buffer which was added to the 9ml of protein (to give a 0.9mg/ml solution). 800ml sodium acetate buffer was purged with N₂ for 15 minutes before the dialysis bag was added. The solution was then purged for a further 2 hours. 2ml thioglycolic acid was added to the buffer which continued to be purged using N₂. After a further 2 hours, another 1ml of thioglycolic acid was added. The buffer was refreshed (800ml pre-purged sodium acetate buffer containing 3ml thioglycolic acid) and dialysis continued under N₂ for 1 hour. The dialysis bag was then transferred to 2 litres PBS at room temp (no N₂) then overnight at 4°C in 3 litres PBS. This demineralised fusion protein was then used to produce paramagnetic fusion protein by the addition of iron and controlled oxidation as below.

### Production of magnetic scFv:ferritin

### Materials

- Reverse osmosis water (RO water)
- 50mM N-(1,1-Dimethyl-2-hydroxyethyl)-3-amino-2-hydroxypropanesulfonic acid (AMPSO) buffer pH8.6 (Sigma A6659)
- 0.1M Sodium acetate buffer pH4.5
- Phosphate buffered saline (PBS) 10mM phosphate, 140mM NaCl pH 7.4
- Trimethylamine-N-oxide (TMA) (Sigma 317594)
- 0.1M ammonium iron (II) sulphate

Trimethylamine-N-oxide (TMA) was heated in an oven to 80°C for 30 minutes to remove Me₃N before cooling to room temperature. 114mg TMA was added to 15ml RO water to produce a 0.07M solution. The iron and TMA solutions were purged with N₂ for 15 minutes before use.

The demineralised fusion protein contained within a dialysis bag (detailed above) was dialysed against 1 litre AMPSO buffer for 2 hour at room temp with stirring under nitrogen. The demineralised scFv:ferritin (∼10ml) was transferred to a conical flask. 18µl iron solution was added to the demineralised protein solution whilst purging with N₂ to scavenge any residual oxygen. After 25 minutes, 15µl iron and 10µl TMA were added.

The following further amounts of iron and TMA buffers were then added at 15 minute intervals:

| | |
|---|---|
| 3^{rd} addition: | 30µl iron + 20µl TMA. |
| 4^{th} addition: | 15µl iron + 10µl TMA |
| 5^{th} addition: | 15µl iron + 10µl TMA |
| 6^{th} addition: | 15µl iron + 10µl TMA |

The magnetised protein was passed through a Macs® LS column. The flow through was passed though a second time to try and increase capture efficiency. The magnetised protein was eluted from the column by removing the column from the magnet and adding 1ml PBS and using the plunger (eluate approx 2ml). This represents a two-fold dilution of the protein on the column.

Eluted protein and controls were coated onto a microtitre plate for analysis as detailed below.

### Analysis of the scFv:magnetoferritin fusion protein by ELISA

In order to ascertain if the magnetised fusion protein retains binding to an anti-ferritin monoclonal antibody an enzyme linked immunosorbant assay was performed.

### Materials

- Carbonate buffer (0.159g sodium carbonate, 03g sodium bicarbonate in 100mls water) pH 9.6
- Phosphate buffered saline (PBS) 10mM phosphate, 140mM NaCl pH 7.4
- Fibronectin peptide
- Anti-fibronectin:ferritin fusion protein (scFv:ferritin)
- Anti-human ferritin murine monoclonal antibody (Santa Cruz SC51887)
- Anti-mouse alkaline phosphatase antibody (Sigma A3562)
- Substrate liquid stable phenolphthalein phosphate
- Stop Solution (212g sodium carbonate, 110.5g 3-(cyclohexylamino)-1-propanesulfonic acid (CAPS), 217g ethylenediamine tetraacetic acid (EDTA) 80g sodium hydroxide, water to 5 litres)
- Maxisorb Microtitre plate (NUNC Cat: 468667)

### Method

### Fusion protein coated wells

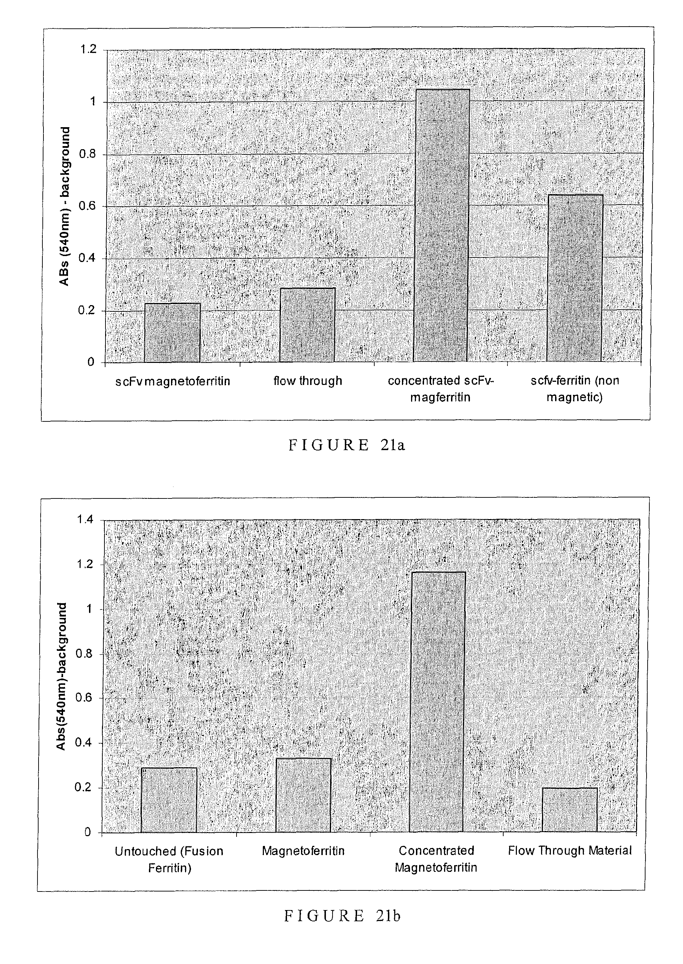

Wells were coated with scFv:ferritin (untouched), scFv:magnetoferritin, scFv:magnetoferritin eluted from the Macs® column and the flow through at a concentration of 1 in 3 in carbonate buffer. The plate was incubated over a weekend at 4°C. Excess solution was flicked off and the plate blocked using 1% BSA in PBS for 1 hour at room temperature. This was flicked off and the plate washed three times using PBS (300 µl/well for each wash). Mouse anti-ferritin antibody was added at a concentration of 20µg/ml and added at a volume of 100µl to each well and incubated at room temperature for an hour. This was removed and the wells washed as before. Goat anti-mouse AP conjugated antibody was diluted to 10µg/ml and added at a volume of 100µl to all wells. This was incubated at room temperature for an hour and removed as before. Substrate was added to all wells and incubated at room temperature for an hour and the reaction stopped using stop buffer. Absorbances were recorded using a Varioskan Flash instrument (Thermo Fisher Electron) (see Figure 21a).

### Fibronectin coated wells

Wells of a microtitre plate were coated with 100µl fibronectin peptide (supplied at 1.5mg/ml) diluted in carbonate buffer to 15µg/ml. The plate was incubated overnight at 2-8°C. Excess solution was flicked off and the wells washed three times in 300µl PBS. The scFv:ferritin fusion proteins were added neat to the appropriate wells (100µl) in duplicate. The plate was then incubated for an hour at room temperature. The solution was flicked off and the wells washed three times in 300µl PBS. Mouse anti-ferritin antibody was added at a concentration of 20µg/ml and added at a volume of 100µl to each well and incubated at room temperature for an hour. This was removed and the wells washed as before. Goat anti-mouse AP conjugated antibody was diluted to 10µg/ml and added at a volume of 100µl to all wells. This was incubated at room temperature for an hour and removed as before. Substrate was added to all wells and incubated at room temperature for 45 minutes and the reaction stopped using stop buffer. Absorbances were recorded using a Varioskan Flash instrument (Thermo Fisher Electron) (see Figure 21b).

The Macs® columns have concentrated the magnetised fusion protein and it is still recognised by the monoclonal anti-ferritin antibody, indicating that the anti-fibronectin-ferritin fusion protein has been magnetised and retained structural integrity. The data also indicates that the magnetised anti-fibronectin ferritin fusion protein retains binding ability to its target antigen and thus illustrates a bi-functional single chain fusion protein that is both magnetisable and can bind a target selectively.

### EXAMPLE 4 - Further Protocols

### Magnetisation of scFv MT2 fusion protein

The scFv-MT2 fusion proteins may be magnetised by replacing zinc ions with manganese and cadmium ions. Methods to do this may be optimised as required. The methods to achieve this include the depletion of zinc by dialysis followed by replacement, also using dialysis with adaptations of published protocols if required.

In detail, these protocols are as follows:
1. Dissolve 5mg MT2 in 5ml buffer (4.5M urea, 10mM Tris base, 0.1M dithiothreitol (DTT), 0.1% mannitol and 0.5mM Pefabloc, pH 11) to strip the protein of the metal ions.
2. Dialyse in the same buffer for 1 hours.
3. Refold the protein by dialysing in buffer 1 (10mM tris base, 2M urea, 0.1M DTT, 0.1% mannitol, 0.5uM Pefabloc and 1mM Cd²⁺/Mn²⁺ pH 11) for 72 hours.
4. Change dialysis buffer to buffer 2 (as above but with urea at a concentration of 1M) and dialyse for 24 hours.
5. Change the dialysis buffer to a buffer as above containing no urea. Dialyse for 24 hours.
6. Change the dialysis buffer as in step 5 to a buffer with pH 8.8. Dialyse for 24 hours.
7. Change the buffer as in step 6 to a buffer containing no mannitol and dialyse as before.
8. Change the buffer as in step 7 to a buffer containing no Cd²⁺/Mn²⁺ and dialyse for 24 hours.

The binding characteristics may be assessed as above in Example 2 for the ferritin fusion protein.

### Protocol for assaying an analyte in a microfluidic device, using the fusion protein

A desired quantity of the fusion protein is mixed with a crude plasma sample containing an analyte of interest within a microfluidic device.

The analyte of interest is trapped along the magnetisable side of the microfluidic device as contaminants are washed away. The magnet is switched off and the purified protein moved to a detection system.

In particular, the invention as described herein provides the following:
1. A label for an analyte, which label is attached to a magnetic or magnetisable substance, the label comprising:
   (a) a recognition moiety for attaching the label to the analyte; and
   (b) a moiety for binding or encapsulating the magnetic or magnetisable substance;
   wherein the moiety for binding or encapsulating the magnetic or magnetisable substance comprises a metal-binding protein, polypeptide, or peptide.
2. A label according to clause 1, which label contains a fusion protein comprising the recognition moiety and the moiety for binding or encapsulating the magnetic or magnetisable substance.
3. A label according to clause 1 or clause 2, wherein the moiety for binding or encapsulating the magnetic or magnetisable substance comprises a protein, or a metal-binding domain of a protein, selected from lactoferrin, transferrin, ferritin, a ferric binding protein, frataxin, a siderophore and a MT.
4. A label according to any preceding clause, which label binds or encapsulates a quantity of the substance having a volume of not more than 1x10⁵ nm³.
5. A label according to clause 4, wherein the moiety for binding or encapsulating the magnetic or magnetisable substance binds or encapsulates a quantity of the substance having a volume of not more than 1x10³ nm³.
6. A label according to clause 5, wherein the moiety for binding or encapsulating the magnetic or magnetisable substance binds or encapsulates a quantity of the substance having a volume of not more than 100 nm³.
7. A label according to any preceding clause, wherein the moiety for binding or encapsulating the magnetic or magnetisable substance is capable of binding transition and/or lanthanide metal atoms and/or ions and/or a compound comprising such ions.
8. A label according to clause 7, wherein the transition metal and/or lanthanide ions comprise any one or more ions of Fe, Co, Ni, Mn, Cr, Cu, Zn, Cd, Y, Gd, Dy, or Eu.
9. A label according to clause 8, wherein the one or more metal ions comprise any one or more of Fe²⁺, Fe³⁺, Co²⁺ Co³⁺, Mn²⁺, Mn³⁺, Mn⁴⁺, Ni²⁺, Zn²⁺ and Cd²⁺.
10. A label according to any preceding clause, comprising a plurality of moieties for binding or encapsulating the magnetic or magnetisable substance, and/or a plurality of recognition moieties.
11. A label according to any preceding clause, wherein the recognition moiety is capable of binding to an analyte selected from a natural or synthetic biological molecule, an infectious agent or component of an infectious agent, a cell or cellular component, and a small molecule.
12. A label according to clause 11, wherein the analyte comprises a virus or virus particle or virus component, a protein, a polypeptide, a glycoprotein, a nucleic acid, such as DNA or RNA, an oligonucleotide, a metabolite, a carbohydrate such as a complex carbohydrate, a lipid, a fat, or an endogeneous or exogeneous small molecule such as a pharmaceutical or drug.
13. A label according to clause 11 or clause 12, wherein the recognition moiety is selected from an antibody or a fragment of an antibody, a receptor or a fragment of a receptor, a protein, a polypeptide, a nucleic acid, and an aptamer.
14. A label according to clause 13, wherein the recognition moiety is selected from a variable polypeptide chain of an antibody (Fv), a T-cell receptor or a fragment of a T-cell receptor, avidin, streptavidin, and heparin.
15. A label according to clause 14, wherein the recognition moiety is selected from a single chain of a variable portion of an antibody (sc-Fv).
16. A label for an analyte, which label is capable of attachment to a magnetic or magnetisable substance, the label comprising:
   (a) a recognition moiety for attaching the label to the analyte, wherein the recognition moiety is an antibody or a fragment of an antibody; and
   (b) a moiety for binding or encapsulating the magnetic or magnetisable substance;
   wherein the moiety for binding or encapsulating the magnetic or magnetisable substance is not ferritin, and comprises a metal-binding protein, polypeptide, or peptide.
17. A label according to clause 16, which label contains a fusion protein comprising the recognition moiety and the moiety for binding or encapsulating the magnetic or magnetisable substance.
18. A label according to clause 16 or clause 17, wherein the moiety for binding or encapsulating the magnetic or magnetisable substance comprises a protein, or a metal-binding domain of a protein, selected from lactoferrin, transferrin, a ferric binding protein, frataxin, a siderophore and a Metallothionein (MT).
19. A label according to any of clauses 16-18, wherein the moiety for binding or encapsulating the magnetic or magnetisable substance is as defined in any of clauses 3-10.
20. A label for an analyte according to any of clauses 16-19 wherein the recognition moiety is a moiety as defined in any of clauses 11-15.
21. A label for an analyte, which label is capable of attachment to a magnetic or magnetisable substance, the label comprising:
   (a) a recognition moiety for attaching the label to the analyte wherein the recognition moiety is not an antibody or a fragment of an antibody; and
   (b) a moiety for binding or encapsulating the magnetic or magnetisable substance;
   wherein the moiety for binding or encapsulating the magnetic or magnetisable substance comprises a metal-binding protein, polypeptide, or peptide.
22. A label according to clause 21, which label contains a fusion protein comprising the recognition moiety and the moiety for binding or encapsulating the magnetic or magnetisable substance.
23. A label according to clause 21 or clause 22, wherein the moiety for binding or encapsulating the magnetic or magnetisable substance comprises a protein, or a metal-binding domain of a protein, selected from lactoferrin, transferrin, a ferric binding protein, frataxin, a siderophore and a MT.
24. A label according to any of clauses 21-23, wherein the moiety for binding or encapsulating the magnetic or magnetisable substance is as defined in any of clauses 3-10.
25. A label for an analyte according to any of clauses 21-24, wherein the recognition moiety is a moiety as defined in any of clauses 11-13.
26. A label bound to an analyte, which label is a label as defined in any of clauses 1-25.
27. A label bound to an analyte according to clause 26, wherein the analyte is an analyte selected from a natural or synthetic biological molecule, an infectious agent or component of an infectious agent, a cell or cellular component, and a small molecule.
28. A label bound to an analyte according to clause 27, wherein the analyte comprises a virus or virus particle or virus component, a protein, a polypeptide, a glycoprotein, a nucleic acid, such as DNA or RNA, an oligonucleotide, a metabolite, a carbohydrate such as a complex carbohydrate, a lipid, a fat, or an endogeneous or exogeneous small molecule such as a pharmaceutical or drug.
29. A method for forming a label as defined in any of clauses 1-28, which method comprises joining a recognition moiety for attaching the label to the analyte, to a moiety for binding or encapsulating a magnetic or magnetisable substance, wherein the moiety for binding or encapsulating the magnetic or magnetisable substance comprises a metal-binding protein, polypeptide, or peptide.
30. A method according to clause 29, wherein the moiety for binding or encapsulating the magnetic or magnetisable substance comprises a protein, or a metal-binding domain of a protein, selected from lactoferrin, transferrin, a ferric binding protein, frataxin, a siderophore and a MT.
31. A method according to clause 29 or clause 30, wherein the moiety for binding or encapsulating the magnetic or magnetisable substance is as defined in any of clauses 3-10.
32. A method for an analyte according to any of clauses 29-31, wherein the recognition moiety is a moiety as defined in any of clauses 11-15.
33. A method of processing a sample, which method comprises:
   (a) contacting the sample with a label for an analyte, which label is attached to a magnetic or magnetisable substance, the label comprising:
      - a recognition moiety for attaching the label to the analyte; and
      - a moiety for binding or encapsulating the magnetic or magnetisable substance;
   (b) subjecting the label to a magnetic field to influence the label;
   (c) optionally analysing the label and/or the analyte to obtain information on an analyte that may be attached to the label.
   wherein the moiety for binding or encapsulating the magnetic or magnetisable substance comprises a metal-binding protein, polypeptide, or peptide.
34. A method according to clause 33, wherein the label contains a fusion protein comprising the recognition moiety and the moiety for binding or encapsulating the magnetic or magnetisable substance.
35. A method according to clause 33 or clause 34, wherein the moiety for binding or encapsulating the magnetic or magnetisable substance comprises a protein, or a metal-binding domain of a protein, selected from lactoferrin, transferrin, a ferric binding protein, frataxin, a siderophore and a MT.
36. A method according to any of clauses 33-35, wherein the moiety for binding or encapsulating the magnetic or magnetisable substance is as defined in any of clauses 3-10.
37. A method for an analyte according to any of clauses 33-36, wherein the recognition moiety is a moiety as defined in any of clauses 11-15.
38. A method according to any of clauses 33-37, wherein the magnetic field is employed to separate, purify and/or isolate the label, and/or an analyte that may be attached to the label, from one or more further substances in the sample.
39. A method according to any of clauses 33-38, in which analysing the label and/or the analyte comprises detecting the presence, absence, identity and/or quantity of the label and/or the analyte.
40. A method according to any of clauses 33-39, which method is carried out using a fluidic device.
41. A method according to clause 40, wherein the fluidic device is a microfluidic device or a nanofluidic device.
42. Use of a moiety for binding or encapsulating a magnetic or magnetisable substance, in a method carried out using a microfluidic or a nanofluidic device, wherein the moiety for binding or encapsulating the magnetic or magnetisable substance comprises a metal-binding protein, polypeptide, or peptide.
43. Use of a label for an analyte, which label is attached to a magnetic or magnetisable substance, the label comprising:
   - a recognition moiety for attaching the label to the analyte; and
   - a moiety for binding or encapsulating the magnetic or magnetisable substance;
   wherein the moiety for binding or encapsulating the magnetic or magnetisable substance comprises a metal-binding protein, polypeptide, or peptide, in a method carried out using a microfluidic or nanofluidic device.
44. Use according to clause 43, wherein the label contains a fusion protein comprising the recognition moiety and the moiety for binding or encapsulating the magnetic or magnetisable substance.
45. Use according to clause 43 or clause 44, wherein the recognition moiety is a moiety as defined in any of clauses 11-15.
46. Use according to any of clauses 42-45, wherein the moiety for binding or encapsulating the magnetic or magnetisable substance comprises a protein, or a metal-binding domain of a protein, selected from lactoferrin, transferrin, a ferric binding protein, frataxin, a siderophore and a MT.
47. Use according to any of clauses 42-46, wherein the moiety for binding or encapsulating the magnetic or magnetisable substance is as defined in any of clauses 3-10.
48. A metal binding fusion protein, comprising one or more recognition agents conjugated to one or more metal-binding proteins, or metal binding domains of such proteins.
49. A fusion protein according to clause 48, comprising one or more variable domains of an antibody genetically conjugated to one or more of either a ferritin or a metallothionein II protein.
50. A fusion protein according to clause 49, wherein the antibody is expressed as a single chain Fv (scFv).
51. A fusion protein according to clause 49 or clause 50, wherein the antibody is a murine antibody.
52. A fusion protein according to any of clauses 48-51 comprising a sequence of SEQ ID 1.
53. A fusion protein according to any of clauses 48-52 comprising a sequence of SEQ ID 2.

## Claims

1. A method of processing a sample, which method comprises:
(i) contacting the sample with a label for an analyte, which label is attached to a magnetic or magnetisable substance;
(ii) subjecting the label to a magnetic field to separate, purify and/or isolate the label, and/or an analyte that may be attached to the label, from one or more further substances in the sample;
(iii) optionally analysing the label and/or the analyte to obtain information on an analyte that may be attached to the label;
wherein the label comprises (a) a recognition moiety for attaching the label to the analyte, and (b) a moiety for binding or encapsulating the magnetic or magnetisable substance comprising a metal-binding protein or a metal binding domain of a metal-binding protein, said metal-binding protein being ferritin.

2. A method according to claim 1 wherein:
(a) analysing the label and/or the analyte comprises detecting the presence, absence, identity and/or quantity of the label and/or the analyte; and/or
(b) the label contains a fusion protein comprising the recognition moiety and the moiety for binding or encapsulating the magnetic or magnetisable substance; and/or
(c) the method is carried out in a fluidic device, preferably a microfluidic or a nanofluidic device.

3. A method according to claim 1 or claim 2 wherein:
(a) the magnetic or magnetisable substance comprises a transition and/or lanthanide metal atoms and/or ions and/or a compound comprising such ions, preferably wherein the transition and/or lanthanide ions comprise any one or more of Fe, Co, Ni, Mn, Cr, Cu, Zn, Cd, Y, Gd, Dy and/or Eu, and more preferably comprises ions of Fe²⁺, Fe³⁺, Co²⁺, Co³⁺, Mn²⁺, Mn³⁺, Mn⁴⁺, Ni²⁺, Zn²⁺ and/or Cd²⁺; and/or
(b) the moiety for binding or encapsulating the magnetic or magnetisable substance binds or encapsulates a quantity of the magnetic or magnetisable substance having a volume of not more than 1x10⁵ nm³, preferably not more than 1x10³nm³, and most preferably not more than 100nm³; and/or
(c) the label comprises a plurality of moieties for binding or encapsulating the magnetic or magnetisable substance, and/or a plurality of recognition moieties.

4. A method according to any one of claims 1 to 3, wherein the recognition moiety is capable of binding to an analyte selected from a natural or synthetic biological molecule, an infectious agent or component of an infectious agent, a cell or cellular component, and a small molecule, and preferably wherein the analyte comprises a virus or virus particle or virus component, a protein, a polypeptide, a glycoprotein, a nucleic acid, such as DNA or RNA, an oligonucleotide, a metabolite, a carbohydrate such as a complex carbohydrate, a lipid, a fat, or an endogeneous or exogeneous small molecule such as a pharmaceutical or drug.

5. A method according to claim 4, wherein the recognition moiety is selected from an antibody or a fragment of an antibody, a receptor or a fragment of a receptor, a protein, a polypeptide, a nucleic acid, and an aptamer, preferably wherein the recognition moiety is selected from a variable polypeptide chain of an antibody (Fv), a T-cell receptor or a fragment of a T-cell receptor, avidin, streptavidin, and heparin, and most preferably wherein the recognition moiety is a single chain of a variable portion of an antibody (sc-Fv).

6. A set of labels for labelling a plurality of analytes, which labels are attached to a magnetic or magnetisable substance, each label in the set comprising:
(i) a recognition moiety for attaching the label to an analyte, and
(ii) a moiety for binding or encapsulating the magnetic or magnetisable substance comprising a metal-binding protein or a metal binding domain of a metal-binding protein, said metal-binding protein being ferritin,
and wherein each label in the set has a unique number of metal-binding moieties.

7. A set of labels according to claim 6 wherein the labels and/or the magnetic or magnetisable substance are as defined in any one of claims 2 to 5.

8. A label for an analyte, which label is attached to a magnetic or magnetisable substance, the label comprising:
(a) a recognition moiety for attaching the label to the analyte; and
(b) a moiety for binding or encapsulating the magnetic or magnetisable substance comprising a metal-binding protein, or a metal-binding domain of a protein, the protein being ferritin,
wherein the magnetic or magnetisable substance comprises ions of Fe, Co, Ni, Mn, Cr, Cu, Zn and/or Cd, and preferably ions of Fe²⁺ Fe³⁺, Co²⁺, Co³⁺, Mn²⁺, Mn³⁺, Mn⁴⁺, Ni²⁺, Zn²⁺ and/or Cd²⁺;
and wherein the label contains a fusion protein comprising the recognition moiety and the moiety for binding or encapsulating the magnetic or magnetisable substance.

9. A label for an analyte according to claim 8 wherein:
(a) the label is as defined in any one of claims 2 to 5; and/or
(b) the label is suitable for use in a method of processing a sample according to claim 1; and/or
(c) the label is part of a set of labels according to claim 6 or claim 7.

10. A fluidic device for performing the method of claim 1, comprising a label for an analyte, which label is attached to a magnetic or magnetisable substance; wherein the label comprises:
(a) a recognition moiety for attaching the label to the analyte, and
(b) a moiety for binding or encapsulating the magnetic or magnetisable substance comprising a metal-binding protein or a metal binding domain of a metal-binding protein, said metal-binding protein being ferritin.

11. A fluidic device according to claim 10 wherein the label and/or the magnetic or magnetisable substance is as defined in any one of claims 2 to 5, and/or wherein the fluidic device is a microfluidic or a nanofluidic device.

12. A fluidic device according to claim 10 or claim 11 which comprises a set of labels according to claim 6 or claim 7.

13. A label bound to an analyte, which label is as defined claim 8 or claim 9, or wherein the label is comprised in a set of labels according to claim 6 or claim 7, wherein the analyte is preferably selected from a natural or synthetic biological molecule, an infectious agent or component of an infectious agent, a cell or cellular component, and a small molecule, and more preferably comprises a virus or virus particle or virus component, a protein, a polypeptide, a glycoprotein, a nucleic acid, such as DNA or RNA, an oligonucleotide, a metabolite, a carbohydrate such as a complex carbohydrate, a lipid, a fat, or an endogenous or exogenous small molecule such as a pharmaceutical or drug.

14. A method for forming a label, wherein the label is as defined in claim 8 or claim 9, or wherein the label is comprised in a set of labels according to claims 6 to 7, which method comprises joining the recognition moiety for attaching the label to the analyte to the moiety for binding or encapsulating the magnetic or magnetisable substance.

15. Use of a label for an analyte in a method carried out in a microfluidic or nanofluidic device, which label is attached to a magnetic or magnetisable substance, the label comprising:
(a) a recognition moiety for attaching the label to the analyte; and
(b) a moiety for binding or encapsulating the magnetic or magnetisable substance,
wherein the moiety for binding or encapsulating the magnetic or magnetisable substance comprises a metal-binding protein, or a metal-binding domain of a metal-binding protein, the metal-binding protein being ferritin.

16. A metal binding fusion protein, comprising one or more recognition agents conjugated to a metal-binding protein, or a metal binding domain of such a protein, wherein the metal-binding protein is ferritin, and wherein the one or more recognition agents are strepavidin or avidin.
